# EUROPEAN PATENT SPECIFICATION

(11) **EP 4 323 059 B1**
(45) Date of publication and mention of the grant of the patent: **28.10.2026**
(21) Application number: 22788876.5
(22) Date of filing: 13.04.2022
(51) Int. Cl.: A61N 1/36, A61B 5/06, A61N 1/00, A61N 1/04, A61N 1/05, A61N 1/372

(54) **SPINAL CORD STIMULATION FOR CONDITIONING RESPIRATORY MUSCLES**
RÜCKENMARKSSTIMULATION ZUR KONDITIONIERUNG DER ATEMMUSKULATUR
STIMULATION DE LA MOELLE ÉPINIÈRE POUR CONDITIONNEMENT DE MUSCLES RESPIRATOIRES

(30) Priority: 14.04.2021 US 202163174979 P
(43) Date of publication of application: 21.02.2024
(73) Proprietor: The Regents of University of California, Oakland, CA 94607-5200 (US)
(72) Inventor: LU, Daniel C., Oakland, California 94607-5200 (US)
(74) Representative: Mewburn Ellis LLP
(86) International application number: PCT/US2022/024673
(87) International publication number: WO 2022/221442

(56) References cited:
- WO-A1-2018/009831
- US-A1- 2003 093 131
- US-A1- 2010 185 253
- US-A1- 2018 008 826
- US-A1- 2018 117 334
- US-A1- 2018 185 642
- US-A1- 2019 192 852
- US-A1- 2019 381 313
- US-A1- 2020 368 523

## Description

### TECHNICAL FIELD

This invention relates generally to the field of spinal cord stimulation for improving respiratory function.

### BACKGROUND

Thousands of patients annually in the United States suffer from respiratory insufficiency or failure, which may be caused by a wide range of conditions such as disease or injury. For example, acute respiratory distress syndrome (ARDS) is a potentially fatal form of respiratory failure that affects approximately 200,000 patients annually in the United States, resulting in nearly 75,000 deaths per year. Approximately 10% of intensive care unit (ICU) admissions are ARDS patients globally, representing more than 3 million patients annually. The best method of dealing with such respiratory failure is invasive mechanical ventilation (e.g., ventilation via an endotracheal tube or tracheostomy with breaths delivered by a mechanical ventilator) to augment deficiencies in oxygenation. However, while largely effective, mechanical ventilation can be a source of significant complication and is not sufficient for avoiding morbidity and mortality. Importantly, under prolonged intubation, patients typically experience ventilator-induced diaphragm dysfunction and/or other atrophy of respiratory musculature that has been measurable within 1-2 days of start of mechanical ventilation. This loss of respiratory function prevents expeditious weaning and removal from mechanical ventilation, which increases ventilation-associated complications and reduces quality of life. Furthermore, prolonged intubation occupies limited hospital resources, thereby limiting access to care, which can be devastating during times of great need for mechanical ventilation equipment, as evidenced by the recent COVID-19 pandemic

WO2018/009831 discloses a system for controlling muscles in a patient.

### SUMMARY

The invention is defined by the independent claim 1. Generally, in some variations, a non-claimed method for conditioning respiratory muscles in a patient may include administering a stimulation signal to one or more of a cervical, thoracic, and lumbar spinal cord of the patient, where the stimulation signal is effective to augment and/or sustain the activation of one or more respiratory muscles in the patient, thereby maintaining strength of the one or more respiratory muscles. In some variations, the spinal cord stimulation may be combined with a cortical stimulation relevant to one or more respiratory muscles. In some variations, the method may include detecting an inspiratory phase of the patient from one or more sensors and administering the stimulation signal during the detected inspiratory phase. In some variations, the method may further include detecting an expiratory phase of the patient from one or more sensors and ceasing the administration of the stimulation signal during the detected expiratory phase. The thoracic spinal cord stimulation may be configured to activate the one or more respiratory muscles by activating motor neurons at a segmental spinal cord level. In some variations, the stimulation signal may be administered to a dorsal column of the thoracic spinal cord. In some variations, the method may include administering a second stimulation signal to a cervical spinal cord region of the patient, where the second stimulation signal is effective to activate respiratory drive in the patient. In some variations, the method may include administering a third stimulation signal to a lumbar spinal cord region of the patient, where the third stimulation signal is effective to activate respiratory drive in the patient.

In some variations, a system for conditioning muscles may include a controller configured to detect an inspiratory phase of the patient based on a sensor signal from one or more sensors, and a stimulator configured to administer a stimulation signal to one or more of a cervical, thoracic, and lumbar spinal cord of the patient during the detected inspiratory phase. The stimulation signal may be effective to activate one or more respiratory muscles in the patient during the inspiratory phase, thereby maintaining strength of the one or more respiratory muscles. In some variations, the spinal cord stimulation may be combined with a cortical stimulation relevant to one or more respiratory muscles. In some variations, the system may include a second stimulator configured to administer a second stimulation signal to a cervical spinal cord of the patient, where the second stimulation signal is effective to activate respiratory drive in the patient. In some variations, the system may include a third stimulator configured to administer a third stimulation signal to a lumbar spinal cord region of the patient, where the third stimulation signal is effective to activate respiratory drive in the patient.

### BRIEF DESCRIPTION OF THE DRAWINGS

FIG. 1A depicts a flowchart illustrating an example variation of a method for conditioning respiratory muscles in a patient.
FIG. 1B depicts diaphragm EMG and respiratory pressure tracings demonstrating diaphragm activation during inspiratory phase of respiration in a subject.
FIG. 1C depicts an illustrative set of signals indicating airway pressure, chest excursion assessed by chest belt, a spinal cord stimulation signal synchronized with inspiration and expiration phase as determined from chest excursion, and a paraspinal EMG signal measuring resultant stimulation from the stimulation signal.
FIG. 2 depicts a schematic of an example variation of a system for conditioning muscles through spinal cord stimulation.
FIG. 3A depicts an experimental setup for an example study investigating the effect of cervical spinal cord epidural stimulation in reversing opioid-induced respiratory depression in human subjects. The spinal level is indicated on the side of the spinal fixation devices, and a stimulating electrode directly contacts the dorsolateral surface of the spine.
FIG. 3B illustrates two stimulation protocols followed in the study related to FIG. 3A.

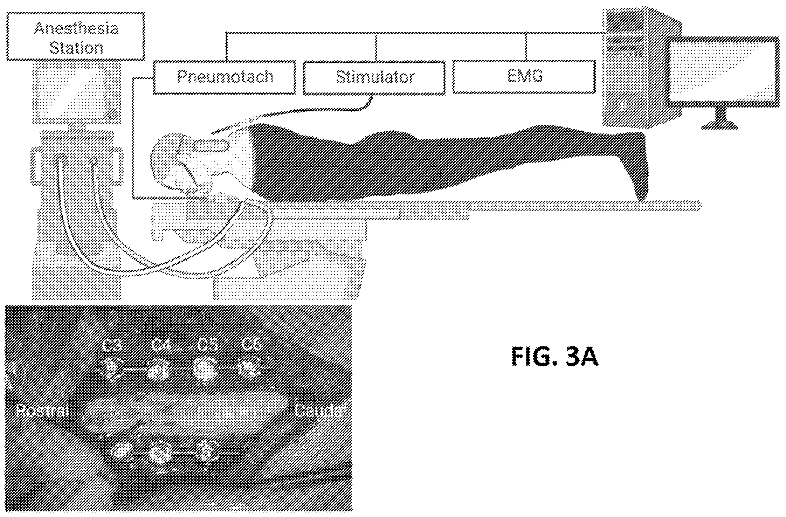

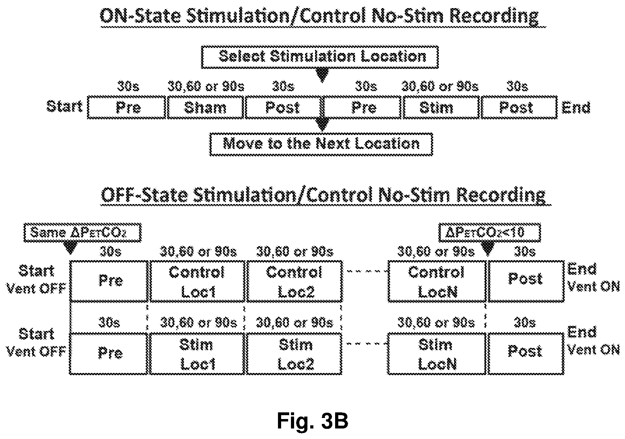

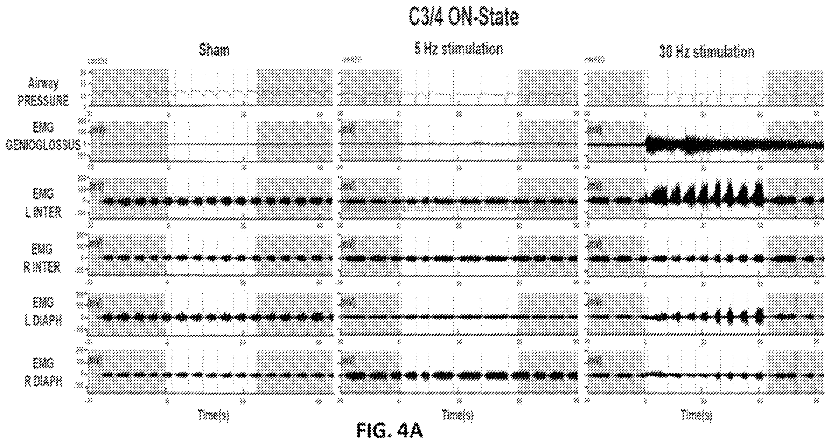

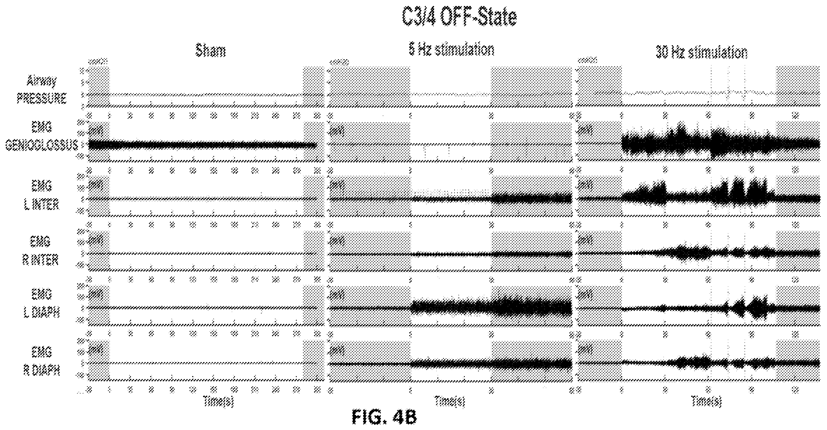

FIGS. 4A and 4B depict control and experimental epidural stimulation at C3/C4 during the example study related to FIGS. 3A and 3B. Specifically, FIG. 4A illustrates representative traces of EMG activity of the genioglossus, left and right intercostal muscles, and left and right sides of the diaphragm in an ON-State (spontaneous, voluntary breathing in subjects). FIG. 4B illustrates representative traces of EMG activity of the genioglossus, left and right intercostal muscles, and left and right sides of the diaphragm in an OFF-State (complete inhibition of spontaneous respiratory activity induced by remifentanil).

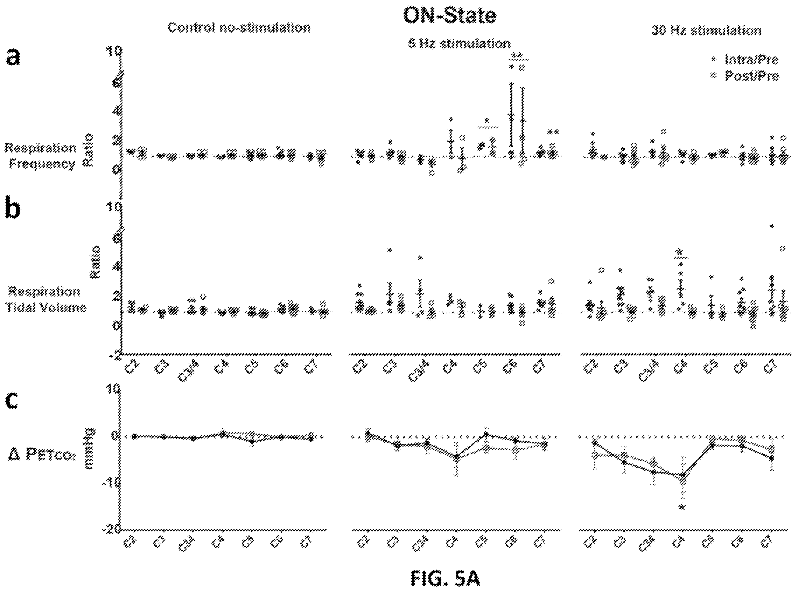

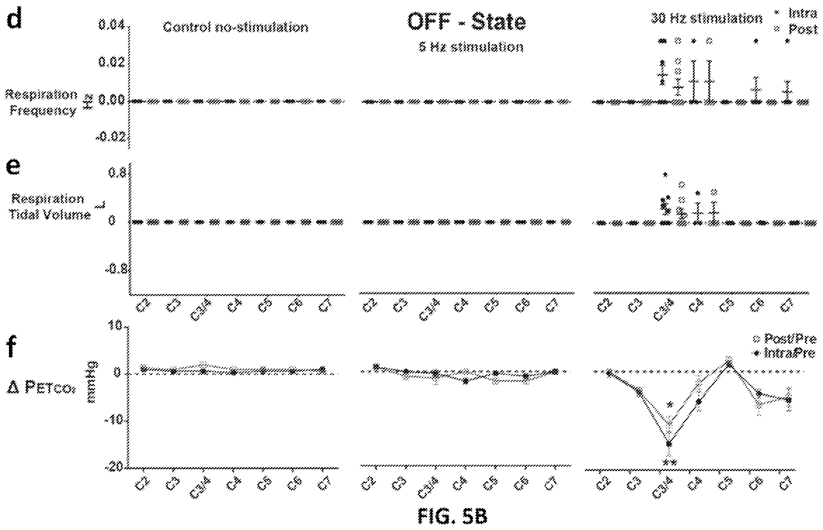

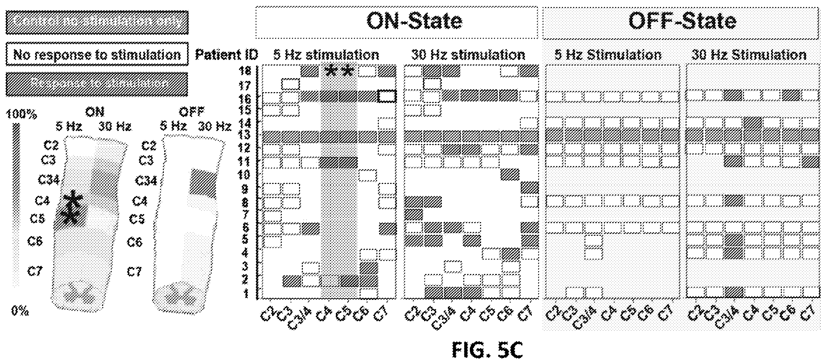

FIG. 5A depicts epidural electrical stimulation (EES) at different cervical levels that induced respiratory pattern changes in the ON-State in the example study related to FIGS. 3A and 3B. Specifically, FIG. 5A depicts changes in respiratory frequency (a), tidal volume (b), and end-title PCO₂ (c) induced by EES at cervical spinal level (C2-C7) during the ON-State. Solid-filled dots represent the outcome during stimulation tests and open-filled dots represent the outcome after control or stimulation tests. Values of respiratory frequency and tidal volume of the ON-State are expressed as the ratio of the respiratory frequency or tidal volume measured in the intra-stim or post-stim conditions to the respective baseline condition. Thus, a non-response equals 1.0. End-tidal PCO₂ is expressed as a change in mm Hg from the baseline value measured during the pre-stimulation condition. The average values and standard error of the mean of all the subjects are shown. "*" indicates where the average response was significantly different from the corresponding control, no-stimulation condition (p < 0.05), while "**" indicates where the p-value <0.01.
FIG. 5B depicts EES at different cervical levels that induced respiratory pattern changes in the OFF-State in the example study related to FIGS. 3A and 3B. Specifically, FIG. 5B depicts changes in respiratory frequency (d), tidal volume (e), and end-tidal PCO₂ (f) expressed as functions of the spinal level stimulated during the OFF-State. Solid-filled dots represent the outcome during stimulation tests and open-filled dots represent the outcome after control or stimulation tests. There was no respiratory frequency and tidal volume during the OFF-State before stimulation, and therefore, a non-response equals 0. End-tidal PCO₂ is expressed as a change in mm Hg from the baseline value measured during the pre-stimulation condition. The average values and standard error of the mean of all the subjects are shown. "*" indicates where the average response was significantly different from the corresponding control, no-stimulation condition (p < 0.05), while "**" indicates where the p-value <0.01.
FIG. 5C depicts a graphical summary of the respiratory responses (Number of patient responses / Total number of patients tested * 100%) at different stimulation sites in the example study related to FIGS. 3A and 3B.

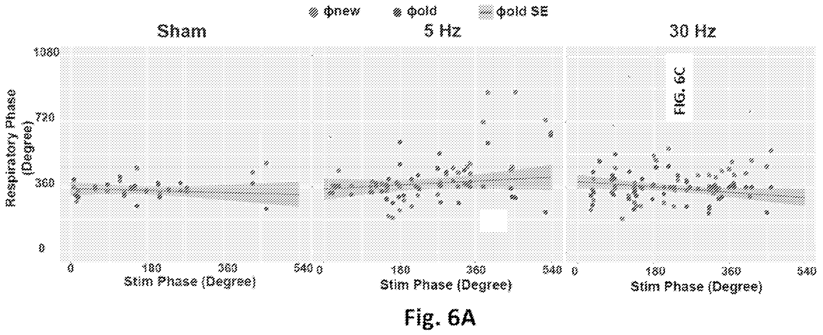

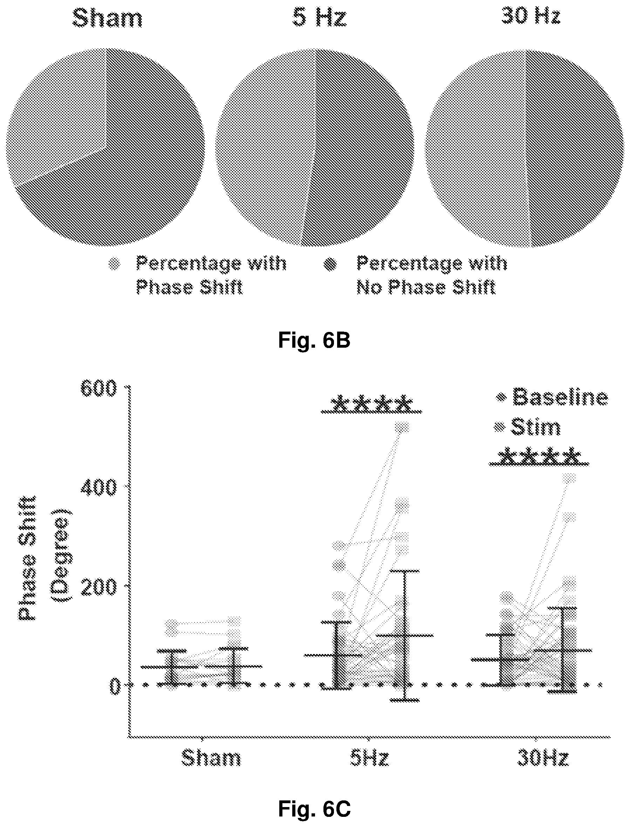

FIGS. 6A-6C depict respiratory oscillatory phase shifts induced by cervical EES in the example study related to FIGS. 3A and 3B. Specifically, FIG. 6A depicts a phase transition curve of the phase comparison between the old phase before the stimulation and the new phase after the stimulation. The 95% confidence interval of the new and old phase when no stimulation was delivered is shown to indicate the spontaneous variation of phase angles in the absence of resetting. FIG. 6B illustrates percentage of the responses with phase shift and percentage of the responses with no phase shift, out of the total tested cases with Sham, 5 Hz, or 30 Hz stimulation. FIG. 6C depicts a comparison of the paired spontaneous phase shift and the stimulation/sham induced phase shift. Repeated measures two-way ANOVA and multiple comparisons with paired t-test was applied to analyze the difference between the phase-shift induced by intervention to the spontaneous phase-shift observed during the pre-stimulation baseline. The mean and the standard error of the mean (SEM) were plotted. **** p<0.0001.

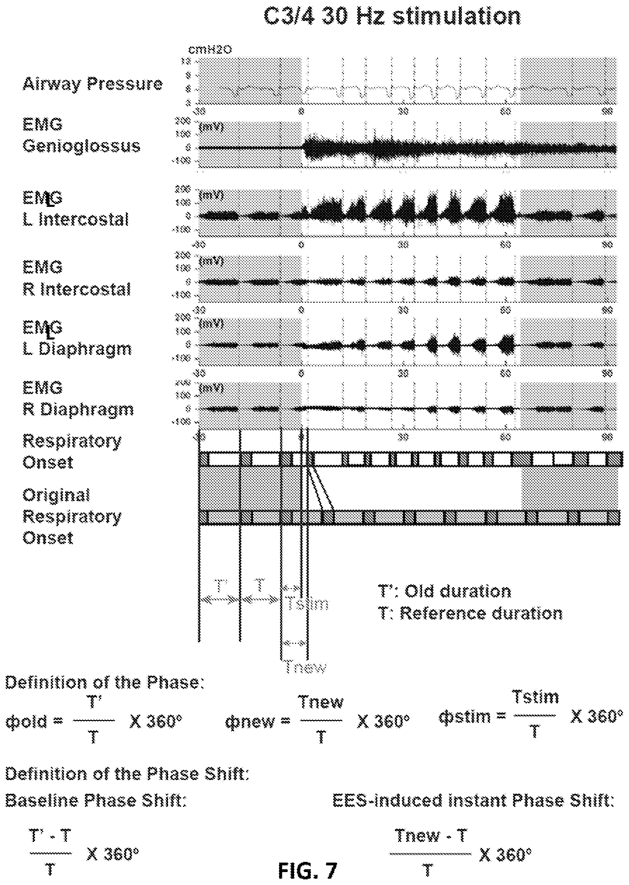

FIG. 7 illustrates the definition of respiratory phase resetting as referenced with respect to FIGS. 6A-6C. FIG. 7 depicts representative traces of tidal pressure ("Airway Pressure") and EMG of the genioglossus, left and right intercostals, and left and right sides of the diaphragm ("EMG Genioglossus", "EMG L Inter", "EMG R Inter", "EMG L Diaph", and "EMG R Diaph", respectively). Below these traces is a time-aligned representation of the inspiratory phase shift after the termination of EES ("Respiratory Onset"), where the dark bars indicate the inspiratory phase and white spaces indicate the expiratory phase. The original, control respiratory phases are shown below the actual respiratory phases for comparison ("Original Respiratory Onset Phases").

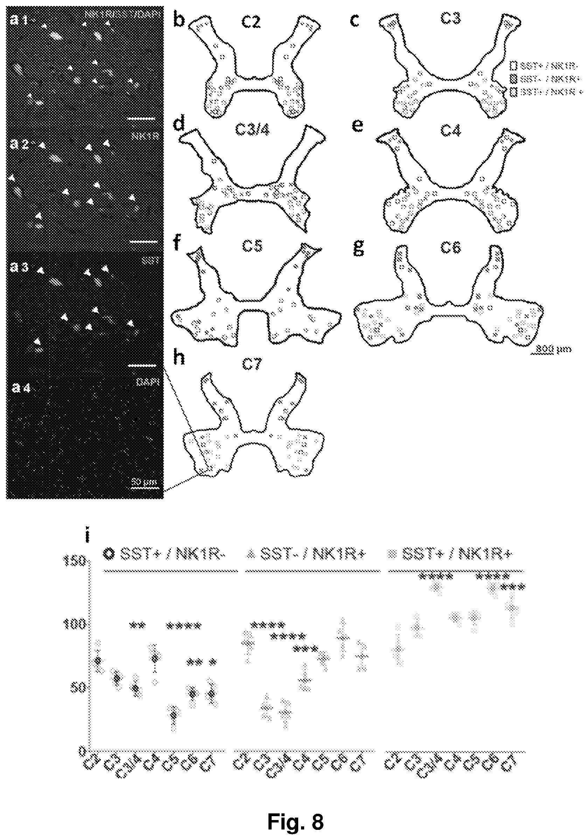

FIG. 8 depicts combined cell distribution maps from five subjects showing SST+/NKIR+ cells in an example study. Specifically, at (panels a1-a4), FIG. 8 depicts photos of NK1R and SST double-positive cells in a sample of spinal cord tissue shown at 40x magnification, including NK1R expression, SST expression, location of cell nuclei, and SST+/NK1R+ expression. Arrows are superimposed to indicate cell body position. At (panels b-h), FIG. 8 depicts 2-D mapping matrices of seven spinal levels averaged among five subjects. At (panel i), FIG. 8 depicts a histogram of SST+/NK1R+, SST+/NK1R-, and SST-/NK1R+ cell counts (*, p<0.05; **, p<0.01; ***, p<0.005, ****, p<0.0001).
FIGS. 9A-9C depict muscle evoked potential (EVP) induced by non-invasive transcutaneous electrical stimulation (TES) to access respiratory spinal motor neurons in an example study.
FIGS. 10A-10D illustrate exemplary results of modifying respiratory response in patients with transcutaneous electrical stimulation in an example study.
FIG. 11 illustrates transcutaneous electrical stimulation at C2/3 inducing coordinated spontaneous respiratory activity in anesthetized humans in an example study.
FIG. 12 illustrates transcutaneous electrical stimulation inducing spontaneous respiratory activity in patient states without pre-existing respiration, via central pattern generation of respiratory muscles.
FIG. 13 illustrates transcutaneous electrical stimulation inducing activation of respiratory muscles through stimulation providing respiratory central pattern generation.
FIG. 14 illustrates transcutaneous electrical stimulation preserving muscle integrity in a mechanically ventilated patient through stimulation providing respiratory central pattern generation.
FIG. 15 illustrates tidal volume heat maps for epidural electrical stimulation (EES) respiratory responses in mice providing respiratory central pattern generation.
FIG. 16 illustrates frequency heat maps for epidural electrical stimulation (EES) respiratory responses in mice providing respiratory central pattern generation.
FIG. 17 illustrates minute ventilation heat maps for epidural electrical stimulation (EES) respiratory responses in mice providing respiratory central pattern generation.

### DETAILED DESCRIPTION

Non limiting examples of various aspects and variations of the invention are described herein and illustrated in the accompanying drawings.

Described herein are methods and systems for spinal cord stimulation for conditioning respiratory muscles, such as to prevent, delay, or treat respiratory muscle atrophy in patients. The spinal cord stimulation may, for example, be administered to patients who are on mechanical ventilation (e.g., acute, chronic), in order to help prevent respiratory muscle atrophy in such patients. Mechanical ventilation may unload (e.g., decrease the force muscles need to generate ventilation) respiratory muscles and lead to muscle atrophy as force generation by muscles at least maintains muscle mass. After intubation and initiation of mechanical ventilation, many patients need large amounts of sedation to tolerate low tidal volume (lung protective) ventilation. The need for sedation (e.g., opioids, propofol) often suppresses the patient's own respiratory muscle activity (e.g., reduces respiratory drive, paralyzes respiratory muscles), which may lead to rapid onset of diaphragm atrophy, which in turn can cause or contribute to delayed weaning and delayed liberation from mechanical ventilation because of respiratory muscle weakness. However, patients on mechanical ventilation who are administered spinal cord stimulation such as that described herein may benefit from maintained or strengthened respiratory muscles, which may reduce time of mechanical ventilation and/or reduce ventilator-associated complications. For example, spinal cord stimulation (e.g., CPG) may induce respiratory activity (with ensemble of respiratory muscles activated including upper airway muscles such as genioglossus, hypoglossal, etc.) when there is no activity and increase frequency of respiration when there is existing respiratory activity. Furthermore, the methods and systems described herein may condition respiratory muscles to expedite the process of weaning from the mechanical ventilation, which reduces patient dependence on and/or reduces duration of mechanical ventilation. In turn, this increases availability of treatment of more patients with mechanical ventilation thereby improving access to care and maximizing valuable hospital resources.

Although in some variations the spinal cord stimulation may be administered to patients who are intubated on a mechanical ventilator or similar assistive equipment as described above, the spinal cord stimulation may be administered to patients in other settings. For example, in some variations, spinal cord stimulation such as that described herein may be administered to a patient who is not intubated on a mechanical ventilator, and the spinal cord stimulation may be administered to delay or prevent the need for mechanical ventilation. In some variations, spinal cord stimulation such as that described herein may be administered to a hospitalized patient to reduce or prevent skeletal and/or respiratory muscle atrophy due to bedrest, or to a patient in an ICU setting to reduce or prevent skeletal and/or respiratory muscle atrophy due to bedrest and/or mechanical ventilation. In some variations, patients in rehabilitation (e.g., at hospital, at home) may receive spinal cord stimulation such as that described herein, to reduce or prevent skeletal and/or respiratory muscle atrophy, promote muscle recovery, and/or the like. As another example, in some variations, spinal cord stimulation such as that described herein may be administered to a patient on venovenous extracorporeal membrane oxygenation (vv ECMO) where the respiratory muscles are not active due to lack of respiratory drive (pCO₂). In some variations, ECMO may serve as a bridge therapy for patients with severe heart and respiratory failure prior to and following surgery (e.g., lung transplant). For example, respiratory muscles may be severely atrophied after a lung transplant and may benefit from spinal cord stimulation as described herein.

The methods and system described herein may be used to treat any suitable patient, such as patient having a respiratory insufficiency or failure (e.g., any condition that requires acute or chronic mechanical ventilation, where respiratory muscles are not active). For example, the respiratory insufficiency may be caused at least in part by respiratory distress syndrome (ARDS, COVID ARDS), ECMO (e.g., vv ECMO), ventilator-induced diaphragm dysfunction, critical illness myopathy, chronic obstructive pulmonary disease (COPD), stroke, spinal cord injury, heart failure, trauma, pneumonia, sepsis, aging, a neurodegenerative disorder (e.g., associated with Parkinson's disease, Alzheimer's disease, Huntington's disease, amyotrophic lateral sclerosis (ALS), congenital central hypoventilation syndrome (CCHS), primary lateral sclerosis (PLS), dystonia, cerebral palsy, Guillain Barre Syndrome, chronic inflammatory polyneuropathy, etc.), or any combination thereof.

### Methods for spinal cord stimulation

In some variations, a method for conditioning respiratory muscles may include administering a stimulation signal to a cervical spinal cord, a thoracic spinal cord, and/or a lumbar spinal cord of a patient, where the stimulation signal is effective to augment and/or sustain the activation of respiratory muscles in the patient, thereby maintaining and/or improving strength of the respiratory muscles. The stimulation may be configured to activate motor neurons, nerve roots and/or interneurons at targeted spinal cord levels for respiratory muscles to prevent muscle atrophy. For example, the stimulation may function to activate local neural circuits that elicit segmental motor responses in intercostal muscles and/or diaphragm. Such stimulation may include subthreshold stimulation (i.e., stimulation that does not reach the necessary threshold for firing and thus does not directly trigger an action potential) and supratheshold stimulation.

One or more strategies may be used to help prevent muscle atrophy. For example, the cervical spinal cord, thoracic spinal cord, and/or lumbar spinal cord stimulation may result in segmental activation of respiratory muscles to condition the respiratory muscles, without interfering with mechanical ventilation (if present) (e.g., the activation of neuro-respiratory substrates specific to diaphragm muscles). As another example, the cervical spinal cord, thoracic spinal cord, and/or lumbar spinal cord stimulation may result in activation of a wide array of respiratory muscles through medullary respiratory central pattern generation (CPG) (e.g., more global, CPG-dependent respiratory muscle activation).

The spinal cord stimulation may be administered to any spinal cord region. For example, in some variations the method may include administering stimulation to a thoracic spinal cord region selected from the group consisting of T7-T7, T7-T8, T7-T9, T8-T8, T8-T9, T9-T9, and T1-11. These thoracic spinal cord regions are associated with segmental activation of respiratory muscles including intercostal (e.g., external intercostal muscle) and diaphragm muscles. Generally, in some variations, the thoracic spinal cord stimulation signal may have a stimulation frequency of at least about 20 Hz, between about 20 Hz and about 100 Hz, between about 1 Hz and about 50 Hz, between about 1 Hz and about 30 Hz, between about 1 Hz and about 10 Hz, or between about 1 Hz and about 5 Hz. In some variations, the stimulation frequency is about 1 Hz or about 2 Hz. Stimulation frequencies of up to about 5 Hz may correspond to segmental stimulation while higher frequencies may access CPG activity regardless of stimulation site. In some variations, segmental stimulation at the respiratory musculature motor pool may be configured to condition muscles at supramotor threshold stimulation. In some variations, the stimulation described herein may be administered during an inspiratory phase.

Additionally or alternatively, the spinal cord stimulation may be administered to a suitable cervical spinal cord region. For example, in some variations the method may include administering stimulation to a cervical spinal cord region selected from the group consisting of C1-C1, C1-C2, C1-C3, C1-C4, C2-C2, C2-C3, C2-C4, C3-C3, C3-C4, C4-C4, C3, C4, C5. These cervical spinal cord regions are also associated with segmental activation of respiratory muscles including intercostal and a primary inspiratory muscle (e.g., diaphragm muscles). Generally, in some variations, the cervical spinal cord stimulation signal may have a stimulation frequency ranging from about 1 Hz, or from about 2 Hz, or from about 3 Hz, or from about 4 Hz, or from about 5 Hz, or from about 10 Hz, or from about 10 Hz, or from about 10 Hz, up to about 500 Hz, or up to about 400 Hz, or up to about 300 Hz, or up to about 200 Hz up to about 100 Hz, or up to about 90 Hz, or up to about 80 Hz, or up to about 60 Hz, or up to about 40 Hz, or from about 3 Hz or from about 5 Hz up to about 80 Hz, or from about 5 Hz to about 60 Hz, or up to about 30 Hz, or of at least about 20 Hz, between about 20 Hz and about 100 Hz, or between about 1 Hz and about 5 Hz. In some variations, the cervical spinal cord stimulation has a stimulation frequency of about 30 Hz. In some variations, the method may include administering stimulation to a cervical spinal cord region at C3/4 at a frequency of at least about 20 Hz. In some variations, the cervical spinal cord stimulation may be similar to that described below with respect to CPG activation. For example, the cervical spinal cord stimulation may be administered for muscle conditioning and/or CPG activation. In some variations, the stimulation described herein may be administered during an inspiratory phase.

Additionally or alternatively, the spinal cord stimulation may be administered to a suitable lumbar spinal cord region. For example, in some variations the method may include administering stimulation to a lumbar spinal cord region selected from the group consisting of L1-L2, L3-L4, and L4-L5. Generally, in some variations, the lumbar spinal cord stimulation signal may have a stimulation frequency of at least about 20 Hz, between about 20 Hz and about 100 Hz, between about 1 Hz and about 50 Hz, between about 1 Hz and about 30 Hz, between about 1 Hz and about 10 Hz, or between about 1 Hz and about 5 Hz.

In some variations of the method, two or more stimulation signals may be administered to a spinal cord region at slightly offset frequencies in a temporal interference (TI) stimulation scheme (e.g., interferential stimulation). For example, cervical, thoracic, and/or lumbar spinal cord stimulation may be performed using two stimulation waveforms that are offset by a frequency difference ranging between about 1 Hz and about 10 Hz, between about 1 Hz and about 5 Hz, or between about 1 Hz and about 2 Hz (e.g., 1 Hz, 2 Hz, 3 Hz, 4 Hz, 5 Hz, 6 Hz, 7 Hz, 8 Hz, 9 Hz, or 10 Hz difference in frequency between the two waveforms). For example, thoracic spinal cord stimulation for conditioning respiratory muscles and/or avoiding respiratory muscle atrophy may be administered by two separate stimulation sources (e.g., electrodes) providing stimulation at 1 Hz and 2 Hz, respectively. As another example, cervical spinal cord stimulation for conditioning respiratory muscles and/or avoiding respiratory muscle atrophy may be administered by two separate stimulation sources (e.g., electrodes) providing stimulation at 30 Hz and 31 Hz (or 5000 Hz and 5001 Hz), respectively. The offset frequencies may induce an interferential stimulation pattern at a predetermined frequency (e.g., about 1 Hz). Without being bound by any particular theory, it is believed that TI stimulation may activate neurons via the offset or beat frequency of the two waveforms, and with reduced stimulation of overlying anatomy not of interest. In some variations, TI stimulation may be applied for transcutaneous spinal stimulation and/or spinal stimulation using needle electrodes. In some variations, the two or more stimulation signals may have a stimulation frequency as described herein of at least about 20 Hz, between about 20 Hz and about 100 Hz, between about 1 Hz and about 50 Hz, between about 1 Hz and about 30 Hz, between about 1 Hz and about 10 Hz, or between about 1 Hz and about 5 Hz.

In some variations of the method, two or more stimulation signals may be administered to a spinal cord region with a first stimulation signal (e.g., monophasic signal, biphasic signal) having a first frequency and an overlapping second stimulation signal having a second frequency higher than the first frequency. For example, cervical, thoracic, and/or lumbar spinal cord stimulation may be performed using two overlapping stimulation waveforms with a first stimulation signal having a frequency between about 0.5 Hz and about 100 Hz, and a second stimulation signal having a frequency between about 5 kHz and about 10 kHz. In some variations, the stimulation may be monopolar or bipolar, and may further comprise an amplitude of between about 0.5 mA and about 200 mA, and a pulse duration of between about 0.5 ms and about 3.0 ms. In some variations, the first stimulation signal may comprise a frequency of at least about 20 Hz, or between about 20 Hz and about 100 Hz, or between about 1 Hz and about 50 Hz, or between about 0.5 Hz and about 30 Hz, or between about 0.5 Hz and about 10 Hz, or between about 0.5 Hz and about 5 Hz. In some variations, the stimulation signal amplitude may range from about 30 mA, or about 40 mA, or about 50 mA, or about 60 mA, or about 70 mA, or about 80 mA up to about 200 mA, or up to about 200 mA, or up to about 150 mA.

In some variations, the method may include combining the cervical spinal cord stimulation, the thoracic spinal cord stimulation, and/or lumbar spinal cord stimulation as described herein with a cortical stimulation relevant to one or more respiratory muscles. The cortical stimulation signal may, for example, help enhance synaptic plasticity in those neural circuits relevant to function of those respiratory muscle(s). For example, in some variations the method may include combining the cervical, thoracic, and/or lumbar spinal cord stimulation described herein with cortical stimulation for the diaphragm muscle, in order to help improve or maintain diaphragm function. The cortical stimulation may be applied to the motor cortex, and the accompanying cervical, thoracic and/or lumbar spinal cord stimulation may be timed to arrive at the cervical, thoracic, and/or lumbar synapses, respectively, substantially simultaneously with (at a predetermined interval after) the cortical stimulation pulse(s) arrive at the cervical, thoracic, and/or lumbar synapses. That is, cortical stimulation to activate affected muscle can be induced to be temporally coincident with spinal stimulation such that a first stimulus administered to a cortical motor neuron arrives at a spinal motor neuron at about the same time as a second stimulus for spinal stimulation arrives at the spinal motor neuron. In some variations, the location of cortical stimulation may be based on (e.g., titrated and adjusted to) a pre-motor or motor cortex homunculus (e.g., cortical representation of associated muscles including the diaphragm).

In some variations, spinal cord associative plasticity may be used to increase volitional motor output. For example, transcranial magnetic stimulation administered in combination with cervical stimulation may increase the force of one or more target muscles. In some variations, cervical transcutaneous spinal cord stimulation pulses administered at subthreshold intensity in combination with magnetic pulses administered over the motor cortex at suprathreshold intensity may be configured to temporally converge in the cervical spinal cord to enhance a hand response. For example, single pairs of cortical-spinal stimulation where a cortical pulse reached the cervical spinal cord up to about 5 milliseconds prior to a spinal pulse enhanced a hand response.

Moreover, stimulation may be administered bilaterally to bilateral muscles or unilaterally to affect a contralateral muscle. In some variations, stimulation may be coordinated to muscle contraction, in the case of autonomic function, such as respiratory function, such that stimulation is active during inspiration. In some variations, cortical stimulation may be administered alone or in combination with spinal stimulation to condition one or more muscles and/or prevent muscle atrophy.

In some variations, cortical stimulation may be configured to activate respiratory muscles through descending inputs to condition one or more muscles through one or more of activation of the CPG, direct or indirect activation of spinal motor neurons (e.g., activation by direct cortical or lumbar to spinal motor neuron pathways or through the respiratory CPG regardless of the site of stimulation; these pathways exist in parallel). In some variations, activation of the CPG may be configured to activate (and strengthen) respiratory muscles with innervation connected through the CPG in proportion to the strength of CPG stimulation. Moreover, direct spinal motor activation may comprise a locally dispersed pattern of stimulation (e.g., thoracic stimulation at T8 may activate motor neurons at T6-12 with a bell-shaped curve of descending activation from the spinal motor neuron activation peak).

In some variations, the spinal cord stimulation may be timed to arrive at the relevant set of synapses at an interval ranging between about 0 ms and about 10 ms after the cortical stimulation pulses arrive at the same. Such an interval may, for example, be about 1 ms, about 2 ms, about 3 ms, about 4 ms, about 5 ms, about 6 ms, about 7 ms, about 8 ms, about 9 ms, or about 10 ms after the arrival of the cortical stimulation pulses. For example, a controller such as the controller 210 described in more detail herein may be configured control a timing of cortical-spinal stimulation as described herein.

The cortical stimulation may be applied transcranially, and may include transcutaneous electrical stimulation, percutaneous electrical stimulation, and/or magnetic stimulation as described in further detail below.

Stimulation of the cervical, thoracic, and/or lumbar spinal cord regions may be performed using a dorsal and/or ventral approach. For example, in some variations, at least one stimulation signal may be administered to a dorsal column of the cervical, thoracic, and/or lumbar spinal cord. A dorsal approach may, for example, be easily accessible and less likely to interfere with other medical equipment such as that associated with a mechanical ventilator.

In some variations, stimulation signals may be administered in a combined dorsal and ventral approach. For example, in a variation in which transcutaneous, percutaneous, or epidural electrical stimulation is administered to a target stimulation location for respiratory muscle conditioning, one or more electrodes may be placed on a posterior region of the patient and one or more electrodes may positioned on an anterior region of the patient. For example, cervical spinal cord transcutaneous, percutaneous, or epidural electrical stimulation for respiratory muscle conditioning may be administered using a posteroanterior configuration in which at least one electrode (e.g., cathode) is positioned over upper thoracic spinous processes, and at least one electrode (e.g., anode) is positioned over an anterior surface of the cervical region of the patient. It is thought, for example, that this posteroanterior electrode configuration may elicit muscle responses across multiple cervical myotomes through sensory afferent and motor efferent circuit activation, where lower stimulation intensities may primarily activate sensor afferent circuits, and higher stimulation intensities may primarily activate motor efferent circuits.

In some variations, a method for conditioning respiratory muscles of a patient may include administering a stimulation signal comprising two alternating pulses of opposite polarities separated by a predetermined delay to form a delayed biphasic pulse waveform. In some variations, the predetermined delay may be up to about 1 µs, between about 1 µs and about 1 µs, between about 1 µs and about 100 µs, or between about 100 µs and about 500 µs. For example, the delayed pulses may comprise a first frequency biphasic carrier pulse (e.g., about 10 kHz) and a second frequency (e.g., about 30 Hz) burst pulse, each pulse having a pulse width of about 1 ms. In some variations, the delayed pulses of the stimulation signal may be applied using transcutaneous electrical spinal cord neuromodulation (TESCoN) (e.g., an adhesive electrode between C3-C4, C5-C6, or T1-T2 serving as the cathode and two adhesive electrodes over bilateral shoulders as the anode.

In some variations, the method may include administering the stimulation signal during one or more treatment sessions. A treatment session may have a duration that ranges, for example, between about 5 minutes and about 30 minutes, between about 5 minutes and about 25 minutes, between about 5 minutes and about 20 minutes, between about 5 minutes and about 15 minutes, between about 10 minutes and about 30 minutes, between about 10 minutes and about 25 minutes, or about 15 minutes.

The patient may undergo multiple treatment sessions, such as periodically at any suitable frequency. For example, the method may include administering the stimulation signal in a treatment session every hour, every 90 minutes, every 2 hours, every 3 hours, or every 5 hours. The repeated treatment sessions may each have the same duration, or they may have different durations. The treatment sessions may be repeated as long as needed to maintain and/or improve respiratory muscle strength. For example, in variations in which the patient is sedated or unconscious and intubated on mechanical ventilation, the treatment sessions may be repeated until the patient regains consciousness. As another example, in variations in which the patient is on bed rest or in rehabilitation, the treatment sessions may be repeated until the patient is no longer on bed rest, or may be repeated throughout a rehabilitation therapy session for multiple rehabilitation therapy sessions until the patient is sufficiently rehabilitated.

In an exemplary variation, a method for conditioning respiratory muscles of a patient includes administering a stimulation signal to a thoracic spinal cord region of the patient in a treatment session every hour, where each treatment session has a duration of about 15 minutes. In variations in which the method is performed on a patient that is sedated, the treatment sessions may be repeated until the patient regains consciousness.

### Stimulation modalities and parameters

The spinal cord stimulation may be administered in a suitable non-invasive manner or invasive manner. For example, in some variations the stimulation signal may be administered transcutaneously, percutaneously, or epidurally. In some variations, the spinal cord stimulation may be electrical or magnetic.

### Transcutaneous electrical stimulation

In some variations, a method of conditioning respiratory muscles may include administering a transcutaneous electrical stimulation signal to a cervical spinal cord region, thoracic spinal cord region and/or lumbar spinal cord region, as described above. The transcutaneous electrical stimulation may, for example, be administered via one or more surface electrodes.

The transcutaneous electrical stimulation signal may have a suitable frequency and amplitude to evoke a motor response of the targeted respiratory muscles (e.g., intercostal muscles, diaphragm). In some variations, the method may include administering to a thoracic spinal cord region a transcutaneous electrical stimulation signal having a stimulation frequency of between about 1 Hz and about 50 Hz, between about 1 Hz and about 30 Hz, between about 1 Hz and about 10 Hz, between about 1 Hz and about 5 Hz, at least about 20 Hz, or between about 20 Hz and about 100 Hz. In some variations, the stimulation frequency is about 1 Hz or about 2 Hz. Additionally or alternatively, in some variations, the method may include administering to a cervical spinal cord region or a lumbar spinal cord region a transcutaneous electrical stimulation signal having a stimulation frequency ranging from about 1 Hz, or from about 2 Hz, or from about 3 Hz, or from about 4 Hz, or from about 5 Hz, or from about 10 Hz, or from about 10 Hz, or from about 10 Hz, up to about 500 Hz, or up to about 400 Hz, or up to about 300 Hz, or up to about 200 Hz up to about 100 Hz, or up to about 90 Hz, or up to about 80 Hz, or up to about 60 Hz, or up to about 40 Hz, or from about 3 Hz or from about 5 Hz up to about 80 Hz, or from about 5 Hz to about 60 Hz, or up to about 30 Hz, at least about 20 Hz, or between about 20 Hz and about 100 Hz. In some variations, the cervical spinal cord or lumbar spinal cord stimulation has a stimulation frequency of about 30 Hz.

Additionally or alternatively, the method may include administering a transcutaneous electrical stimulation signal having an amplitude of between about 5 mA to about 300 mA, or between about 5 mA to about 250 mA, or between about 5 mA to about 200 mA, between about 5 mA to about 150 mA, or between about 5 mA to about 100 mA, or between about 5 mA to about 80 mA, or between about 5 mA to about 60 mA, or between about 5 mA to about 50 mA.

In some variations, the transcutaneous electrical stimulation signal may be superimposed on a high frequency carrier signal. The high frequency carrier signal may, for example, range between about 3 kHz and up to about 30 kHz, or up to about 20 kHz, or up to about 15 kHz. In certain embodiments the carrier signal is about 10 kHz. In some variations, the carrier frequency amplitude may range from about 30 mA, or about 40 mA, or about 50 mA, or about 60 mA, or about 70 mA, or about 80 mA up to about 300 mA, or up to about 200 mA, or up to about 150 mA.

### Percutaneous stimulation

In some variations, a method of conditioning respiratory muscles may include administering a percutaneous electrical stimulation signal to a cervical spinal cord region, a thoracic spinal cord region and/or a lumbar spinal cord region, as described above. The percutaneous stimulation may, for example, be administered via one or more needle electrodes.

The percutaneous electrical stimulation signal may have a suitable frequency and amplitude to evoke a motor response of the targeted respiratory muscles (e.g., intercostal muscles, diaphragm). In some variations, the method may include administering to a thoracic spinal cord region a percutaneous electrical stimulation signal having a stimulation frequency of between about 1 Hz and about 50 Hz, between about 1 Hz and about 30 Hz, between about 1 Hz and about 10 Hz, between about 1 Hz and about 5 Hz, at least about 20 Hz, or between about 20 Hz and about 100 Hz. In some variations, the stimulation frequency is about 1 Hz or about 2 Hz. Additionally or alternatively, in some variations, the method may include administering to a cervical spinal cord region or a lumbar spinal cord region a percutaneous electrical stimulation signal having a stimulation frequency ranging from about 1 Hz, or from about 2 Hz, or from about 3 Hz, or from about 4 Hz, or from about 5 Hz, or from about 10 Hz, or from about 10 Hz, or from about 10 Hz, up to about 500 Hz, or up to about 400 Hz, or up to about 300 Hz, or up to about 200 Hz up to about 100 Hz, or up to about 90 Hz, or up to about 80 Hz, or up to about 60 Hz, or up to about 40 Hz, or from about 3 Hz or from about 5 Hz up to about 80 Hz, or from about 5 Hz to about 60 Hz, or up to about 30 Hz, at least about 20 Hz, or between about 20 Hz and about 100 Hz. In some variations, the cervical spinal cord or lumbar spinal cord stimulation has a stimulation frequency of about 30 Hz.

Additionally or alternatively, the method may include administering a percutaneous electrical stimulation signal having an amplitude of between about 5 mA to about 300 mA, or between about 5 mA to about 250 mA, or between about 5 mA to about 200 mA, between about 5 mA to about 150 mA, or between about 5 mA to about 100 mA, or between about 5 mA to about 80 mA, or between about 5 mA to about 60 mA, or between about 5 mA to about 50 mA.

In some variations, the percutaneous electrical stimulation signal may be superimposed on a high frequency carrier signal. The high frequency carrier signal may, for example, range between about 3 kHz and up to about 30 kHz, or up to about 20 kHz, or up to about 15 kHz. In certain embodiments the carrier signal is about 10 kHz. In some variations, the carrier frequency amplitude may range from about 30 mA, or about 40 mA, or about 50 mA, or about 60 mA, or about 70 mA, or about 80 mA up to about 300 mA, or up to about 200 mA, or up to about 150 mA.

### Epidural stimulation

In some variations, a method of conditioning respiratory muscles may include administering an epidural electrical stimulation signal to a cervical spinal region, a thoracic spinal cord region and/or a lumbar spinal cord region, as described above. The epidural stimulation may, for example, be administered via one or more implanted electrodes placed at an epidural site.

The epidural stimulation signal may have a suitable frequency to evoke a motor response of the targeted respiratory muscles (e.g., intercostal muscles, diaphragm). In some variations, the method may include administering to a thoracic spinal cord region an epidural stimulation signal having a stimulation frequency of between about 1 Hz and about 50 Hz, between about 1 Hz and about 30 Hz, between about 1 Hz and about 10 Hz, between about 1 Hz and about 5 Hz, at least about 20 Hz, or between about 20 Hz and about 100 Hz. In some variations, the stimulation frequency is about 1 Hz or about 2 Hz. Additionally or alternatively, in some variations, the method may include administering to a cervical spinal cord region or a lumbar spinal cord region an epidural electrical stimulation signal having a stimulation frequency ranging from about 1 Hz, or from about 2 Hz, or from about 3 Hz, or from about 4 Hz, or from about 5 Hz, or from about 10 Hz, or from about 10 Hz, or from about 10 Hz, up to about 500 Hz, or up to about 400 Hz, or up to about 300 Hz, or up to about 200 Hz up to about 100 Hz, or up to about 90 Hz, or up to about 80 Hz, or up to about 60 Hz, or up to about 40 Hz, or from about 3 Hz or from about 5 Hz up to about 80 Hz, or from about 5 Hz to about 60 Hz, or up to about 30 Hz, at least about 20 Hz, or between about 20 Hz and about 100 Hz. In some variations, the cervical spinal cord or lumbar spinal cord stimulation has a stimulation frequency of about 30 Hz.

Additionally or alternatively, the method may include administering an epidural stimulation signal having an amplitude of between about 5 mA to about 300 mA, or between about 5 mA to about 250 mA, or between about 5 mA to about 200 mA, between about 5 mA to about 150 mA, or between about 5 mA to about 100 mA, or between about 5 mA to about 80 mA, or between about 5 mA to about 60 mA, or between about 5 mA to about 50 mA.

### Magnetic stimulation

In some variations, a method of conditioning respiratory muscles may include administering a stimulation signal, induced by a magnetic signal, to a cervical spinal region, a thoracic spinal cord region, and/or a lumbar spinal cord region, as described above. The magnetic signal may, for example, be administered via one or more magnetic coils (e.g., magnetic wand).

The stimulation signal induced by a magnetic signal may have a suitable frequency to evoke a motor response of the targeted respiratory muscles (e.g., intercostal muscles, diaphragm). In some variations, the method may include administering to a thoracic spinal cord region a stimulation signal having a stimulation frequency of between about 1 Hz and about 50 Hz, between about 1 Hz and about 30 Hz, between about 1 Hz and about 10 Hz, between about 1 Hz and about 5 Hz, at least about 20 Hz, or between about 20 Hz and about 100 Hz. In some variations, the stimulation frequency is about 1 Hz or about 2 Hz. Additionally or alternatively, in some variations, the method may include administering to a cervical spinal cord region or a lumbar spinal cord region a stimulation signal having a stimulation frequency ranging from about 1 Hz, or from about 2 Hz, or from about 3 Hz, or from about 4 Hz, or from about 5 Hz, or from about 10 Hz, or from about 10 Hz, or from about 10 Hz, up to about 500 Hz, or up to about 400 Hz, or up to about 300 Hz, or up to about 200 Hz up to about 100 Hz, or up to about 90 Hz, or up to about 80 Hz, or up to about 60 Hz, or up to about 40 Hz, or from about 3 Hz or from about 5 Hz up to about 80 Hz, or from about 5 Hz to about 60 Hz, or up to about 30 Hz, at least about 20 Hz, or between about 20 Hz and about 100 Hz. In some variations, the cervical spinal cord or lumbar spinal cord stimulation has a stimulation frequency of about 30 Hz.

In some variations, the stimulation is administered by producing a magnetic field strength of up to about 10 tesla, up to about 8 tesla, up to about 6 tesla, up to about 5 tesla, up to about 4 tesla, up to about 3 tesla, up to about 2 tesla, or up to about 1 tesla.

The stimulation signal may have a suitable frequency and amplitude to evoke a motor response of the targeted respiratory muscles (e.g., intercostal muscles, diaphragm). In some variations, the method may include administering a stimulation signal having a stimulation frequency of between about 1 Hz and about 50 Hz, between about 1 Hz and about 30 Hz, between about 1 Hz and about 10 Hz, or between about 1 Hz and about 5 Hz. In some variations, the stimulation frequency is about 1 Hz or about 2 Hz.

### CPG activation

Respiration involves a complex network of circuits that is involved in central pattern generation (CPG) that spans the brainstem and cervical spinal cord to generate a respiratory rhythm. In some variations, the method may additionally or alternatively include activating the respiratory network to activate respiratory drive by administering stimulation to a cervical spinal cord region of the patient.

Accordingly, in some variations, the method for conditioning respiratory muscles may include administering a second stimulation signal to a cervical spinal cord of the patient, where the second stimulation signal is effective to activate a respiratory drive in the patient. The second stimulation signal may, for example, activate rostrally-directed sensor input to brainstem respiratory circuits that in turn increase widespread activation of respiratory muscles. The second stimulation signal to the cervical spinal cord may, for example, be administered simultaneously with a stimulation signal to the thoracic spinal cord and/or lumbar spinal cord as described above. Together, the cervical, thoracic, and/or lumbar spinal cord stimulation may be performed to more effectively condition the respiratory muscles (e.g., to expedite ventilator weaning).

Similarly, in some variations, the method for conditioning respiratory muscles may include administering a third stimulation signal to a lumbar spinal cord of the patient, where the third stimulation signal is effective to activate a respiratory drive in the patient. The second stimulation signal may, for example, activate rostrally-directed sensor input to brainstem respiratory circuits that in turn increase widespread activation of respiratory muscles. The third stimulation signal to the lumbar spinal cord may, for example, be administered simultaneously with a stimulation signal to the thoracic spinal cord and/or cervical spinal cord as described above. Together, the cervical, thoracic, and/or lumbar spinal cord stimulation may be performed to more effectively condition the respiratory muscles (e.g., to expedite ventilator weaning or where the respiratory muscles are not active due to vv ECMO).

In some variations, administering the third stimulation signal may be performed during the detected inspiratory phase. In some variations, the third stimulation signal may be administered to a region selected from the group consisting of L1-L2, L3-L4, and L4-L5.

The cervical spinal cord stimulation may, in some variations, be similar to that described in U.S. Patent Pub. No. 2018/0185642 entitled "Accessing Spinal Network to Enable Respiratory Function" and/or U.S. Patent Pub. No. 20190381313 entitled "Accessing Spinal Network to Enable Respiratory Function". For example, in some variations, the cervical spinal cord stimulation may be administered to a region selected from the group consisting of C2-C2, C2-C3, C2-C4, C3-C3, C3-C4, and C4-C4. Furthermore, the cervical spinal cord stimulation signal may have a stimulation frequency ranging from about 1 Hz, or from about 2 Hz, or from about 3 Hz, or from about 4 Hz, or from about 5 Hz, or from about 10 Hz, or from about 10 Hz, or from about 10 Hz, up to about 500 Hz, or up to about 400 Hz, or up to about 300 Hz, or up to about 200 Hz up to about 100 Hz, or up to about 90 Hz, or up to about 80 Hz, or up to about 60 Hz, or up to about 40 Hz, or from about 3 Hz or from about 5 Hz up to about 80 Hz, or from about 5 Hz to about 60 Hz, or up to about 30 Hz, at least about 20 Hz, or between about 20 Hz and about 100 Hz. In some variations, the cervical spinal cord stimulation has a stimulation frequency of about 30 Hz.

Like the thoracic and lumbar spinal cord stimulation described above, the cervical spinal cord stimulation may be in the form of transcutaneous electrical stimulation, percutaneous electrical stimulation, epidural stimulation, and/or stimulation induced by a magnetic signal.

### Closed loop stimulation

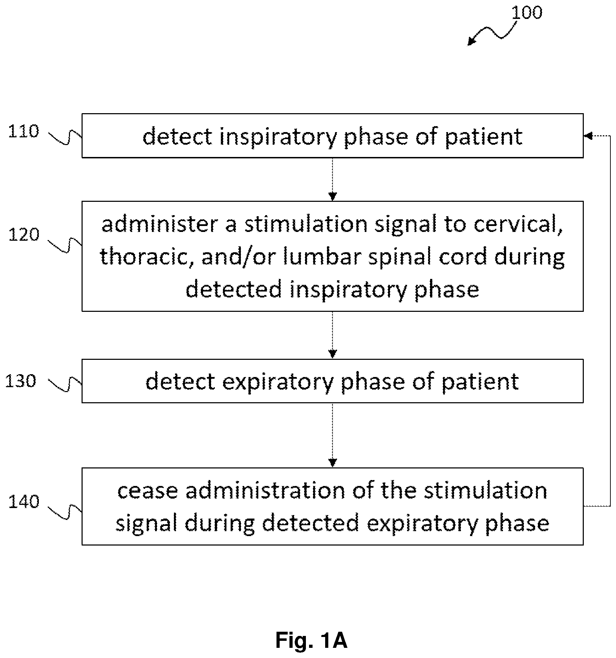

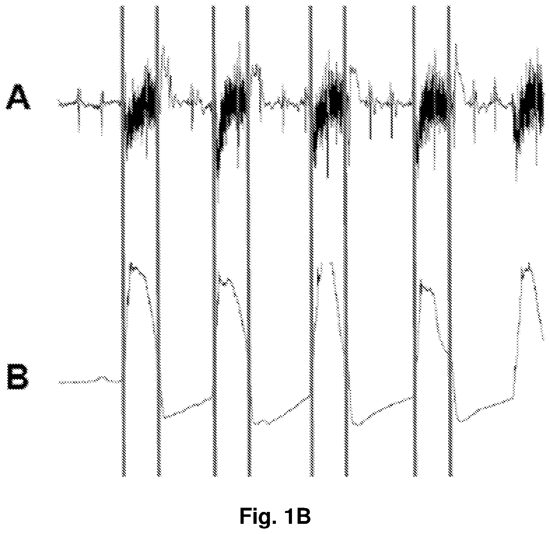

In some variations, the spinal cord stimulation may be administered in a temporally coordinated manner with respiratory activity. For example, to properly condition the respiratory muscles, the method may include stimulating one or more regions of the cervical, thoracic, and/or lumbar spinal cord in phase with an inspiratory phase of the patients. Accordingly, in some variations, as shown in FIG. 1A, a method **100** for conditioning respiratory muscles in a patient includes detecting an inspiratory phase of the patient **110** and administering a stimulation signal to one or more of a cervical, thoracic, and/or lumbar spinal cord of the patient **120** during the detected inspiratory phase. Furthermore, the method **100** may include detecting an expiratory phase of the patient **130** and ceasing administration of the stimulation signal during the detected expiratory phase of the patient **140.**

Similar to that described above, the thoracic and/or lumbar stimulation signal may be effective to augment and/or sustain the activation of one or more respiratory muscles in the patient during the inspiratory phases, thereby maintaining strength of the one or more respiratory muscles. Additionally or alternatively, the cervical stimulation signal may be effective to activate a respiratory drive in the patient. The stimulation signal(s) administered in response to the detected inspiratory phase may be similar to any of the stimulation signals described above.

FIG. 1B illustrates an example respiratory pressure tracing and associated diaphragm EMG with spontaneous respiration in a human subject. Specifically, waveform (B) depicts respiratory pressure tracing across several respiratory cycles, where increased pressure peaks correlate to inspiratory phases of respiration. Waveform (A) depicts EMG tracings (as measured by EMG sensors) of diaphragm muscle. As shown by the alignment of active segments of waveform (A) and the inspiratory phases of waveform (B), the diaphragm muscle is activated during the entire inspiratory phase of respiration. In variations of the method 200 in which cervical and/or thoracic stimulation is performed in a biomimetic closed-loop manner based on detection of inspiratory phase, the conditioning of the diaphragm muscle may be configured to emulate the EMG activity of waveform (A).

The inspiratory phase of the patient may be detected based one or more sensor signals, in that the sensor signal(s) may provide feedback in the closed-loop stimulation method to indicate inspiration, expiration, and/or other respiration activity of the patient. For example, sensor signals from a sensor configured to detect chest wall expansion or other movement and provide a sensor signal indicating the same. As described in further detail below, examples of such sensors include a device attachable to the patient and measures thoracic impedance or rib cage movement (e.g., inductance band, strain gauge band, accelerometer) of the patient. Accordingly, the cervical and/or thoracic spinal cord stimulation may be administered in phase with the inspiratory phase detected based on one or more such sensor signals.

In variations in which the patient is on mechanical ventilation, the cervical, thoracic, and/or lumbar spinal cord stimulation administered to the patient may be coordinated with mechanical ventilation. As further described below, an output signal from the mechanical ventilator indicating ventilation cycle or phase may be provided and analyzed to determine timing of the induced inspiratory phase of the patient. Accordingly, the cervical, thoracic, and/or lumbar spinal cord stimulation may be administered in phase with mechanical ventilator activity.

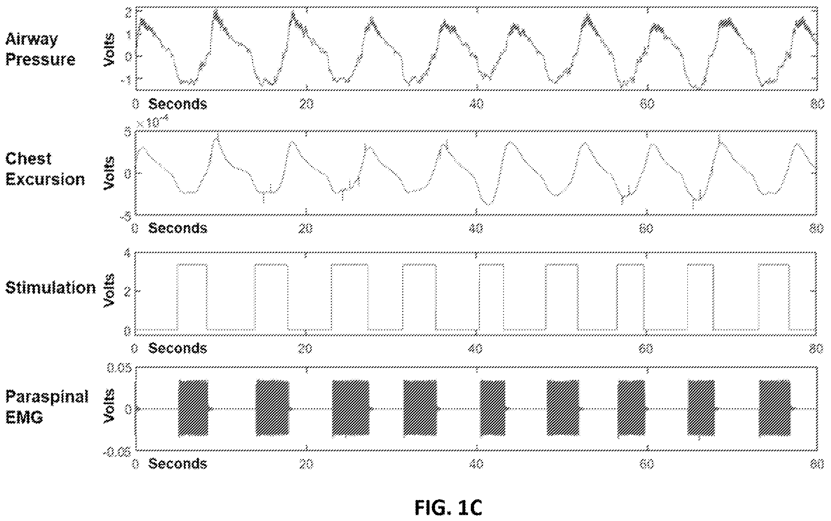

For example, FIG. 1C illustrates an example variation of a method in which closed-loop, transcutaneous electrical spinal cord stimulation is performed in response to chest movement assessed by an optional chest belt. Chest wall movement is captured by a PVDF chest wall sensor ("chest excursion"), whose signal is processed to determine inspiratory phase. Cervical spinal cord stimulation ("stimulation") is administered at a 30 Hz stimulation frequency during initial phase of inspiration. FIG. 1C also illustrates how resultant stimulation may be captured by paraspinal EMG placed next to the stimulation electrode ("paraspinal EMG"). The resultant stimulation is responsive and closed-loop to different respiratory intervals. At the conclusion of inspiration and initiation of expiration, the stimulation may be terminated until the next inspiratory cycle, thereby imparting a closed-loop stimulation system. Although FIG. 1C illustrates stimulation and thresholds of a particular exemplary variation, it should be understood that the threshold of activation and termination of stimulation may be altered in any suitable manner. Furthermore, the closed-loop nature of stimulation may be expanded to apply to other suitable cervical, thoracic, and/or lumbar spinal cord stimulation signals (e.g., modality, various slope of ramp up and down of stimulus, frequency, intensity, pulse width, etc.), such as any of the cervical, thoracic, and/or lumbar spinal cord stimulation modalities or parameters as described above.

### Systems for spinal cord stimulation

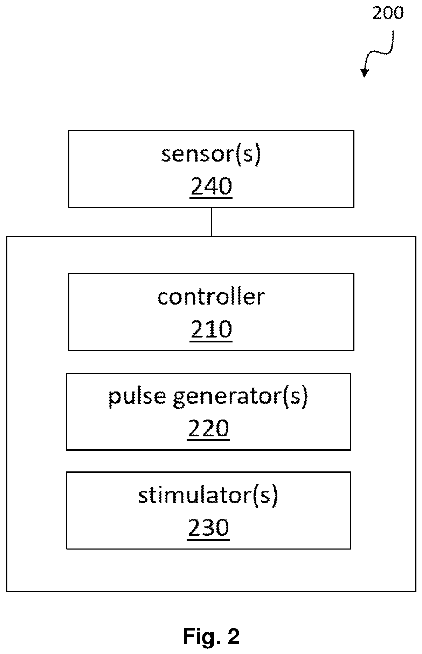

As shown in FIG. 2, a system **200** for spinal cord stimulation for conditioning respiratory muscles may include a controller **210,** at least one pulse generator **220,** and at least one stimulator **230.** The controller **210** may be communicatively coupled to one or more sensors **240** to collect one or more sensor signals, such as a sensor signal indicating inspiration, expiration, and/or other respiratory activity of the patient.

Furthermore, the system **200** may include suitable circuitry elements (not pictured) for providing power to the electronic components of the system **200,** allowing for network connectivity (e.g., data communication), and/or other suitable elements. At least a portion of the system **200** may, in some variations, be implantable. In variations in which at least a portion of the system **200** is implanted, the system **200** may include wireless power transfer elements such as antennas or coils for providing wireless power to implanted electronics (e.g., via inductive coupling, radiative coupling, etc.), or a wired power transfer via conductive connection. In some variations, the system **200** may include a power storage element such as a battery or capacitor. In some variations, the system **200** may comprise a mechanical ventilator configured for one or more of invasive and non-invasive ventilation of a patient.

### Controller

In various embodiments, the controller **210** functions to modulate stimulation pulses to be administered to the patient through the stimulator(s) **230.** For example, the controller **210** may regulate the stimulation parameters produced by the stimulator(s) **230,** and/or control on/off timing of the stimulation provided by the stimulator(s) **230.** The controller **210** may be separate from the stimulator(s) **230,** or may be integrated with one or more of the stimulator(s) **230.** The controller **210** may include or be operably coupled to one or more memory devices storing instructions to control the stimulation signal(s), and may include one or more processors for performing analysis (e.g., analyzing sensor signals), determining instructions to send to the stimulator for signal generation, determining timing of such instructions, and/or the like.

As described above, the controller **210** may be communicatively coupled to one or more sensors **240,** such as to receive one or more sensor signals indicating respiratory activity. The controller **210** may receive such sensor signals, analyze the sensor signals to determine inspiration phase, expiration phase, and/or other suitable features of the patient's respiratory activity, and control the ramping up/ramping down of the stimulation signal(s) and/or modulate stimulation parameters as described above. In some variations, stimulation by the stimulator **230** may be modulated based on a measured respiratory phase of the patient monitored by one or more sensors **240.**

### Sensor(s)

In some variations, the system **200** may include one or more sensors **240** configured to provide sensor signals indicative of respiratory activity. For example, the system **200** may include one or more sensors configured to detect chest wall expansion or other chest movement. Various sensor technologies may be suitable for detecting chest wall expansion or other chest movement. For example, rib cage movement can be measured with an inductance or strain gauge band placed around the rib cage (e.g., immediately below the axillae). In some variations, the one or more sensors may be part of an inductance band, in which chest expansion can be determined by changes in the inductance of the band induced by stretching of the band. Similarly, in some variations, the one or more sensors may be part of a strain gauge band, in which changes in resistance/conductance of the strain gauges produced by band expansion/contraction can readily be measured using methods known to those of skill in the art. As another example, chest wall expansion and/or movement can be monitored with a sensor measuring thoracic impedance. In some variations, respiratory phase may be estimated with a sensor measuring one or more of airway flow and end tidal CO₂.

Additionally or alternatively, a sensor **240** may include an accelerometer attached to the surface of the body (e.g., with a wearable band or other garment, with adhesive, etc.), while in other variations, the accelerometer can be implanted within the body. The signal from the accelerometer may be analyzed to determine respiratory activity by, for example, mapping movement and/or timing of chest wall movements.

### Stimulator

In various embodiments, the system **200** may include one or more stimulators 230 configured to administer stimulation to a spinal cord region. Each stimulator **230** may include a pulse generator and one or more stimulation elements configured to administer stimulation in accordance with pulses generated by the pulse generator. The pulse generator and one or more stimulation elements may be housed together or may be housed separately (and connected to each other by a lead, for example).

In some variations, the stimulation elements of a stimulator **230** may be configured to provide transcutaneous electrical stimulation. The stimulator **230** may, for example, include one or more surface electrodes applicable to skin of the patient proximate the target spinal cord location (e.g., thoracic spinal cord, cervical spinal cord, lumbar spinal cord, etc.). In some variations, the one or more surface electrodes may be adhesive so as to be removably attached to the skin. Alternatively, in some variations, the stimulation elements for providing transcutaneous electrical stimulation may include one or more surface electrodes on a housing (e.g., handheld device, wearable device, etc.) that may be held against or secured to the patient such that the surface electrodes contact the skin.

In some variations, the stimulation elements of a stimulator **230** may be configured to provide percutaneous electrical stimulation. The stimulator **230** may, for example, include one or more needle electrodes that may be inserted at one or more target stimulation locations. For example, a percutaneous lead may include two or more spaced electrodes (e.g., equally or unequally spaced electrodes), that are placed above the dura layer (e.g., through the use of a Touhy-like needle). For insertion, the Touhy-like needle can be passed through the skin, between desired vertebrae, to open above the dura layer.

In some variations, the stimulation elements of a stimulator **230** may be configured to provide epidural stimulation. The stimulator **230** may, for example, include an implanted electrode array. In some variations, the implanted electrode array may be a high density electrode array prepared using suitable microfabrication techniques to place numerous electrodes in an array configuration on a flexible substrate. The electrode arrays may include one or more biocompatible metals (e.g., gold, platinum, chromium, titanium, iridium, tungsten, and/or oxides and/or allow thereof) disposed on a flexible material. Examples of suitable flexible materials include parylene A, parylene C, parylene AM, parylene F, parylene N, parylene D, silicon, and other flexible substrate materials, or combinations thereof. The implanted electrode array may be implanted using any of a number of methods (e.g., a laminectomy procedure), such as those known to those of skill in the art. For example, in some embodiments, electrical energy is delivered through electrodes positioned external to the dura layer surrounding the spinal cord. Stimulation on the surface of the cord (subdurally) is also contemplated, for example, stimulation may be applied to the dorsal columns as well as to the dorsal root entry zone.

In some variations, implanted electrodes can also be provided with an implantable controller **210** and/or an implantable power source. The implantable controller **210** may be programmed/reprogrammed by use of an external device (e.g., using a handheld device that communicates with the control circuitry through the skin). The programming can be repeated as often as necessary.

In some variations, the electrodes for electrical stimulation (transcutaneous, percutaneous, epidural) may be operably linked to the controller **210** that permits selection of electrode(s) to activate/stimulate and/or that controls frequency, and/or pulse width, and/or amplitude of stimulation. In some variations, the electrode selection, frequency, amplitude, and pulse width may be independently selectable, e.g., at different times, different electrodes can be selected. At any time, different electrodes can provide different stimulation frequencies and/or amplitudes. In some variations, different electrodes or all electrodes can be operated in a monopolar mode and/or a bipolar mode, using constant current or constant voltage delivery of the stimulation.

In some variations, the stimulation elements of a stimulator **230** may be configured to provide stimulation induced by a magnetic signal. The stimulator **230** may, for example, include one or more elements configured to generate a magnetic field that induces current at the spinal cord region of interest. In some variations, effective nerve stimulation may be achieved with a current transient of about 10⁸ A/s. In certain variations this current is obtained by switching the current through an electronic switching component (e.g., a thyristor or an insulated gate bipolar transistor (IGBT)).

In some variations, a magnetic stimulator may include a high current pulse generator producing discharge currents (e.g., 5,000 amps or more) and a stimulating coil producing magnetic pulses (e.g., with field strengths up to 4, 6, 8, or even 10 tesla) and with a pulse duration typically ranging from about 100 µs to 1 ms or more, depending on the stimulator type. The stimulating coils may, in some variations, include one or more well-insulated copper windings, together with temperature sensors and safety switches. A voltage (power) source (e.g., a battery) may charge a capacitor or other power storage element via charging circuitry under the control of control circuitry (e.g., a microprocessor) that accepts information such as the capacitor voltage, power set by the user, and various safety interlocks within the equipment to ensure proper operation, and the capacitor may then connected to the coil via an electronic switching component when the stimulus is to be applied. The control circuitry may be operated via a controller interface that can receive user input and/or sensor signal(s) and adjust stimulus parameters in response.

When activated, the discharge current flows through the coils inducing a magnetic flux. It is the rate of change of the magnetic field that causes the electrical current within tissue to be generated, and therefore a fast discharge time is important to stimulator efficiency. Accordingly, an electrical current is generated within the tissue, and that it is the electrical current that causes the depolarization of the cell membrane and thus the stimulation of the target nerve.

Since the magnetic field strength falls off with the square of the distance from the stimulating coil, the stimulus strength is at its highest close to the coil surface. The stimulation characteristics of the magnetic pulse, such as depth of penetration, strength and accuracy, depend on the rise time, peak electrical energy transferred to the coil and the spatial distribution of the field. The rise time and peak coil energy are governed by the electrical characteristics of the magnetic stimulator and stimulating coil, whereas the spatial distribution of the induced electric field depends on the coil geometry and the anatomy of the region of induced current flow.

As described above, in various embodiments the system **200** may include multiple stimulators (or multiple stimulating elements). For example, the system **200** may include a first stimulator configured to administer thoracic spinal cord stimulation, a second stimulator configured to administer cervical spinal cord stimulation, and a third stimulator configured to administer lumbar spinal cord stimulation. In some variations with multiple stimulators, the first, second, and/or third stimulators may be substantially identical in construction (e.g., having same modality, such as both stimulators providing transcutaneous electrical stimulation, percutaneous electrical stimulation, epidural stimulation, or magnetic stimulation). Alternatively, in some variations with multiple stimulators, the first, second, and/or third stimulators may be different (e.g., having different stimulation modalities). Furthermore, it should be understood that the system 200 may include any suitable number of stimulators, such as one, two, three, four, five, or more stimulators.

### Glutamatergic agents

Somatostatin (SST) and neurokinin-1 receptors (NK1R) in the cervical spinal cord are involved in gating afferent inputs and may be associated with the generation of respiratory pattern (i.e., respiratory drive) in the brainstem (see Example 3 below). Glutamate may be a helpful excitatory neurotransmitter for improving the mechanism for SST+/NK1R+ neurons in the cervical spinal cord.

Accordingly, to further enhance respiratory function (e.g., enhance CPG for respiratory drive, improve combined enhancement of respiratory function in a patient some variations, the spinal cord stimulation methods described herein may be used in conjunction with one or more pharmacological agents, such as glutamatergic agents.

These agents can be administered alone or in conjunction with spinal cord stimulation as described herein. For example, glutamatergic agents may be administered in conjunction with transcutaneous electrical stimulation, percutaneous electrical stimulation, epidural electrical stimulation, and/or magnetic stimulation of the cervical spinal cord and/or thoracic spinal cord, as described herein. This combined approach can help to put the spinal cord in an optimal physiological state for activating and/or improving respiratory drive, and/or otherwise enhancing respiratory function (e.g., for conditioning respiratory muscles as described herein).

In some variations, glutamatergic agent(s) may be administered systemically, though additionally or alternatively, glutamatergic agent(s) may be administered locally (e.g., to particular regions of the spinal cord, such as the cervical spinal cord). Glutamatergic agent(s) can be administered or delivered by injection (e.g., subcutaneously, intravenously, intramuscularly), orally, rectally, inhaled, or in any suitable manner.

### Examples

The following examples are offered to illustrate, but not to limit the claimed invention.

### Example 1 - Activation of respiratory centers with EES

Opioid overdose suppresses brainstem respiratory circuits and may result in death. We tested the hypothesis that exogenous stimulation of the cervical spine could activate respiratory centers in the ventral medulla and increase ventilation in humans. To determine if neuromodulation can reverse opioid-induced respiratory depression (OIRD), we used a commercially available electrode approved for intraoperative monitoring to apply epidural electrical stimulation (EES) dorsally at cervical spinal levels C2 to C7 in 17 anesthetized human subjects whose respiration was suppressed following administration of the synthetic opioid analgesic remifentanil. One subject was only tested in the control, no-stimulation condition. We administered two doses of remifentanil to the tested subjects: a low dose, which was 0.01-0.03 mcg/kg/min, and a high dose, which was 0.03-0.1 mcg/kg/min. Low-dose remifentanil partially suppressed respiration. High-dose remifentanil induced respiratory depression that led to apnea.

FIG. 3A illustrates the experimental setup, along with a typical example of the exposed surface of the spine labeled with spina level. The stimulating electrode is shown directly contacting the dorso-lateral surface of the spine. Constant current epidural stimulation was administered using the ES-IX 2 Stimulator (Cadwell, Kennewick, USA) via a disposable double-ball tip direct nerve stimulator probe (Cadwell #302431). A grounding needle was placed at the tissue surface near the surgical incision. Constant current stimulation consisted of square-wave, monophasic pulses at 5 Hz or 30 Hz, each with a 500 µsec pulse-width. The optimal stimulation intensity ranged from 3 mA - 5 mA. Stimulation was applied for 30, 60, or 90 s at each cervical location in the ON-State or OFF-State. We defined the optimal stimulation intensity as 80% of the current required to elicit a minimally-perceptible motor response in the deltoid muscle of either arm. Therefore, all stimulation current levels were below the threshold for deltoid activation. We applied stimulation settings that are within the range commonly used for clinical neurophysiological assessments and safe for use during surgery in patients. The effects of epidural electrical stimulation were compared to the effects of a control no-stimulation procedure (sham). The sham consisted of placing the stimulating electrode on the dorsolateral dural surface of the spinal cord with the same movement and pressure applied during genuine stimulation, but without delivering any electrical current.

We defined spontaneous, voluntary breathing in subjects as the ON-State, whereas complete inhibition of spontaneous respiratory activity (remifentanil-induced apnea) was defined as the OFF-State. We tested seventeen subjects in ON-State and eight of them were tested in both ON- and OFF-States. EMG recordings measured the extent to which EES affected respiratory muscle activity. We assessed two frequencies of constant current, monopolar EES applied to seven locations in subjects in ON- and OFF-States to determine optimal stimulation parameters (FIG. 3B).

5 Hz EES was found to increase respiratory frequency and 30 Hz EES was found to increase amplitude of respiration in patients with voluntary respiration. Among the subjects tested in the ON-State (FIGS. 4A, 5A), 30 Hz EES at the level of the cervical spinal segment 4 (C4) significantly increased respiratory tidal volume (p = 0.0164, Intra/Pre) and decreased the end-tidal carbon dioxide (ΔP_{ETCO2}, p = 0.017, Post/Pre) compared to control, no-stimulation values at the same cervical level in the same subject.

EES stimulation at 5Hz at cervical spinal segments C5, C6, and C7 increased respiratory frequency compared to control values (C5, p=0.0356; C6, p=0.0061; and C7, p=0.0092), but did not significantly increase tidal volume or decrease ΔP_{ETCO2} (FIGS. 5A-5C). However, the increased respiratory frequency induced by 5 Hz stimulation at all three cervical levels persisted after stimulation ceased and decayed over 60-90 s. Given that the P_{ETCO2} and pulse oximeter oxygen saturation (SpO₂) were stable when respiratory frequency changed (indicating no change in chemical drive to breathe), we believe that EES-initiated a short-term potentiation of the respiratory frequency that persistted after the EES ceased, and this potentiation decayed over 60-90 seconds.

The stimulation intensity of EES was below the motor activation threshold. Deltoid muscle EMG activity did not respond to 5- or 30 Hz EES at any cervical level tested in ON-State subjects, including the C5-C6 levels where the motor neurons that innervate the deltoid muscles reside. We observed genioglossal EMG activation in 73% of the tests that elicited a respiratory response, from which we infer that EES activated the hypoglossal nuclei in the brainstem. Moreover, the genioglosssal EMG activity was coordinated with EMG activation of the diaphragm and intercostal muscles and preceded activation of these 'pump muscles', as it typically does during eupnea. These observations indicate that motor neuron pacing was not the root cause of the responses that we measured, but rather that EES of the dorsal cervical spine activated neurons that augmented activity of the endogenous respiratory control system within the brainstem, which in turn increased the respiratory frequency or magnitude of respiratory muscle activation.

**30 Hz stimulation induced sustained respiratory responses when voluntary respiration was depressed by high-dose remifentanil.** Eight out of the seventeen subjects were tested in the OFF-State in the absence of any voluntary respiration (FIG. 5B). Thirty Hz stimulation had its greatest effect between cervical spinal segment levels 3 and 4 (C3/4), where activation of respiratory muscles was apparent in six subjects (75%), and three out of these six subjects continued to breathe after stimulation ceased. We recorded a significant decrease of PETCO2 in these subjects - indicative of effective alveolar gas exchange. In the example OFF-State response in FIG. 4B, genioglossal and left external intercostal EMG activity increased immediately. EMG activity of the intercostal muscles and diaphragm began on the left side, which was the side of the spine that we targeted at C3/4, and as the EES persisted, phasic EMG activation appeared on both sides of the diaphragm. Sequential activation of hypoglossal EMG activity and pump muscle EMG activity was present only after phasic EMG activity developed. This rhythmic respiratory pattern was initiated in five of the six subjects, including all three of the subjects in whom respiratory activation persisted after EES ceased.

Although 5 Hz stimulation in OFF-State subjects marginally decreased PETCO2 after stimulation, it failed to induce meaningful respiratory activity (FIG. 5B). We observed changes in tonic EMG activity of the genioglossal, intercostal, and diaphragm muscles, but no changes in phasic activity were recorded (FIG. 5B (d)). Compared to 5 Hz EES, 30 Hz stimulation was more effective inducing respiratory activation during the OFF-State (FIG. 5G). However, in the ON-State, different cervical spinal cord levels were differentially responsive to 30 Hz or 5 Hz EES. Five Hz EES more frequently yielded greater respiratory responses at C5 - C7, whereas 30 Hz was more effective initiating respiratory responses at more rostral cervical spinal levels (C2-C4) (FIG. 5B).

**Cervical EES reset the respiratory cycle.** A further analysis of the inspiratory onset in the ON-State indicated that cervical EES reset the respiratory cycle, which was evaluated using a phase transition analysis of the respiratory cycle (FIG. 6A). The phase transition was measured by noting the change in phase (or not) when EES was applied at different times of the respiratory cycle. To describe the spontaneous variation of respiratory phase durations, we calculated the old phase (ϕold) as the ratio of the two cycles before stimulation was delivered. The stimulation phase (ϕstim) was defined as the ratio of the interval between the onset of inspiration to the onset of EES divided by the phase duration of the previous unstimulated breath, which defines when in the respiratory cycle EES began. To capture the impact of EES on phase relationships of the stimulated breaths (breath durations), we defined the new phase as the ratio of the stimulated breath duration to the duration of the breath preceding stimulus delivery (ϕnew) (FIG. 7). All ratios were converted to degrees. The new phase ϕnew and the old phase ϕold (ordinate; FIG. 6A) were plotted as functions of the stimulation phase ϕstim (abscissa). To show the effect of spontaneous phase variation during the respiratory cycle without EES, we plotted the 95% confidence interval of ϕold (shaded areas, FIG. 6A); ϕnew that fall outside this range likely represent phase resetting (FIG. 6B). Respiratory cycle resetting was more common during 5 or 30 Hz EES than during sham stimulation (FIGS. 3B and 3C), but resetting may advance or delay the respiratory cycle, and angles are not normally distributed. Therefore, we took the absolute value of the change in phase angles and transformed the absolute values of the change in phase angles using the Box-Cox transformation so that the data were homoscedastic and consistent with the assumptions of the parametric statistical analyses that we used. The EES-induced phase angle changes observed after the start of 5 Hz EES and 30 Hz EES were significantly greater than the spontaneous variation of phase angles in the Sham condition (p <0.0001 for both sham vs. 5 Hz and sham vs 30 Hz, FIG. 3B). This finding is consistent with resetting observed after single pulses of epidural stimulation in which resetting was frequently observed after dorsal cervical EES (but not ventral EES). We infer from this analysis that the cervical neurons or axons of passage activated by EES interact with and resynchronize the respiratory central pattern generator in the brainstem, which is also consistent with the patterns of upper airway and pump muscle activation that we observed during dorsal cervical EES.

Accordingly, we studied the capability of EES to modulate the rhythmic respiratory pattern itself in a setting where communication with the effector motor neurons in the ventral spine were intact, but the activity of the respiratory rhythm/pattern generator was suppressed. We observed that 30 Hz EES at C3/4 augmented the minute tidal volume in patients who were breathing spontaneously and induced cyclic respiratory activity in patients with apnea after remifentanil administration. The effect of the EES persisted after the EES stopped, suggesting that EES of the dorsal cervical spine elicited short-term potentiation of the respiratory pattern generator, which decayed within 60-90 seconds after EES ceased. The cervical spinal cord circuit may be regarded as the effector of respiratory muscle activation downstream from the more rostral, ponto-medullary network generating rhythmic respiratory activity. We observed that cervical EES not only augmented the amplitude of the respiration but also actively modulated the frequency of respiratory oscillations. Five Hz stimulation at C5 to C7 regions increased the respiratory frequency and often induced phase shifting of the respiratory cycle at the onset of EES; whereas 30 Hz stimulation increased the tidal volume during EES at the C4 level. Dual effects of dorsal cervical EES at different locations of the spine suggest that different sets of cervical neurons or axons of passage expressed different frequency-dependent patterns of activation, and that the different sets of activated neurons or axons communicate with different parts of the respiratory central pattern generator that control respiratory frequency (likely more rostral ponto-medullary elements of the respiratory pattern generating network) and tidal volume (likely more ventral medullary elements of the respiratory central pattern generator or spinal phrenic motor neurons).

### Example 2 - Administration of a glutamatergic agent to enhance respiratory function

Characterizing the distribution of cells with somatostatin (SST) and neurokinin-1 receptors (NK1R) in the cervical spinal cord may lead to better understanding of the mechanism of respiratory neuromodulation. Spinal SST+ and NK1R+ neurons are both involved in gating afferent inputs and SST+/NK1R+ neurons are associated with the generation of the respiratory pattern in the brainstem. We conducted an immunohistochemical analysis to identify the distribution of SST+ and NK1R+ neurons in the cervical spinal region in five human cadavers. Levels C3/4, C6, and C7 contained significantly high densities of SST and NK1R, double-positive neurons (FIG. 8) in the lamina layers III through IX (LIII-IX) compared to SST and NK1R staining at the C2 level of the spine. These levels are also the spinal levels most responsive to EES. The correlation of cervical SST+/NK1R+ neurons in post-mortem human tissue samples and responsiveness to EES at the cervical spine in vivo raises the possibility that cervical neurons may have similar neuronal properties to the brainstem neurons that affect respiration. Accordingly, in view of these results, administering a glutamatergic agent to a patient may help enhance respiratory function.

### Example 3 - Segmental activation of respiratory muscles through TES

We investigated non-invasive neuromodulation of the respiratory spinal cord circuit, specifically with transcutaneous electrical stimulation (TES). This spinal neuromodulation has a substantial safety profile; there has been no TES-associated side-effects in combined > 50,000 hours of treatment in more than 50 subjects.

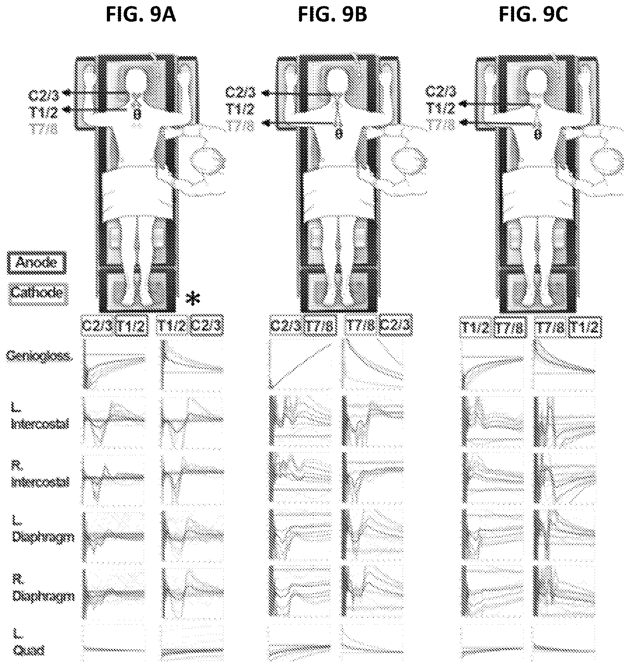

As shown in FIGS. 9A-9C, bilateral, monophasic, 1 Hz transcutaneous electrical stimulation was tested in bipolar configurations of electrode placement (C2/3 + T1/2, C2/3 + T7/8, and T1/2 + T7/8). Each electrode configuration was tested with both directions of polarities (anode and cathode placement). The muscle evoked potentials (EVP) of the respiratory muscles, genioglossus, bilateral intercostal, and bilateral diaphragm muscles were recorded to evaluate the efficiency of the specific electrode configuration in recruiting and accessing the respiratory motor neurons. The muscle EVP of the right lower extremity was recorded as a non-respiratory control. For this representative patient, the configuration of transcutaneous stimulation that induced the most prominent EVP activations (early phase) of the diaphragm and intercostal muscles are highlighted as effective setting (especially C2/3 cathode, T7/8 anode, "*"). In a cohort of 8 patients, T1/2 cathode, C2/3 anode configuration was significantly higher compared to other configurations at 5.375 ±1.43 mV (p<0.05). p <0.0005 by one-way ANOVA, post-hoc Tukey. Criteria for effectiveness is the greatest amplitude of respiratory muscle EVP at lowest intensity of stimulation within 100 ms window of stimulation. Average of 15 EVP were performed and averaged. Patient position shown in FIGS. 9A-9C is for illustration purposes only, as it should be understood that stimulation can be conducted with the patient any suitable position such as in supine, prone, or lateral positions.

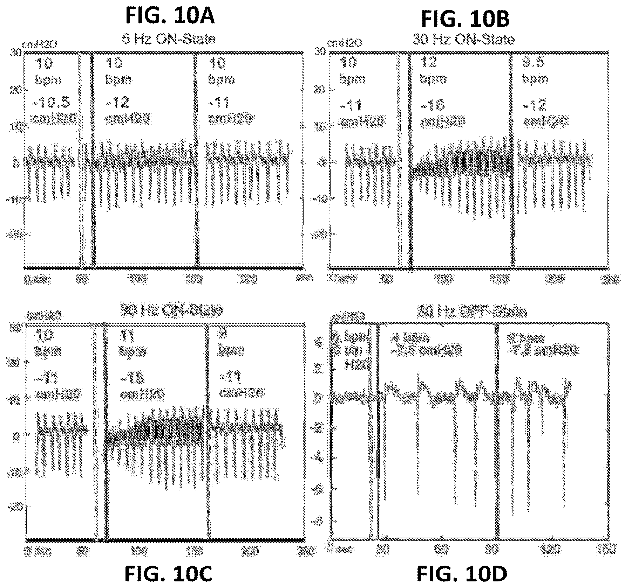

FIGS. 10A-10D illustrate exemplary results of modifying respiratory response in patients with transcutaneous electrical stimulation. TES targeting C2/3 spinal cord modified spontaneous respiration in a representative patient intubated in preparation for elective surgery (A-C, "ON-state") and in another representative patient without spontaneous respiration (D, "OFF-State, 30-Hz shown"). Ventilator setting during recording was switched off with patient breathing through the breathing tube. Respiration frequency in breath per minute (bpm) and airway pressure (cmH20) were measured in three phases: before simulation, during stimulation, and after stimulation. Maximum respiratory frequency and maximum airway pressure were calculated based on the maximum values reached during each phase. Vertical lines demarcate changes made to stimulator: in each of FIGS. 10A-10D, the leftmost line demarcate ramp up of stimulator, the middle line denotes reaching maximum stimulator intensity (100 mA), and the rightmost line denotes switching off of stimulation. Up to 20% and 45% increase in respiratory frequency and airway pressure were observed in the sample patient with preexisting spontaneous respiration, respectively. Comparing the respiratory frequency in ON-state within the cohort of 10 subjects treated with 30-Hz stimulation, before stim 9.3 bpm (±1.77), during stim 11.4 bpm (±1.43), and post stim 8.75 bpm (±1.59) were observed. Before stimulation vs. stimulation, p<0.05; stimulation vs. post-stimulation, p <0.01. p <0.005 by one-way ANOVA, post-hoc Tukey. In patient without spontaneous respiration, respiratory activity can be induced with up to 4 breaths per minute and -7.5 cmH20 during stimulation. Comparing the respiratory frequency within the OFF-state cohort of 10 subjects treated with 30-Hz stimulation, before stim 0 bpm (±0), stimulation 4.5 bpm (±1.96), and post-stimulation (5.9±2.42) bpm were observed. Before stimulation vs. stimulation or post-stimulation, p<0.01. p <0.0005 by one-way ANOVA, post-hoc Tukey. No significant responses were observed in control 5 Hz stimulation in either ON- or OFF-state (p>0.05). Bpm = breaths per minute; Hz = hertz; sec = second. Positive deflection is expiration and negative is inspiration of air, (± standard deviation). Similar results were observed in 10 subjects.

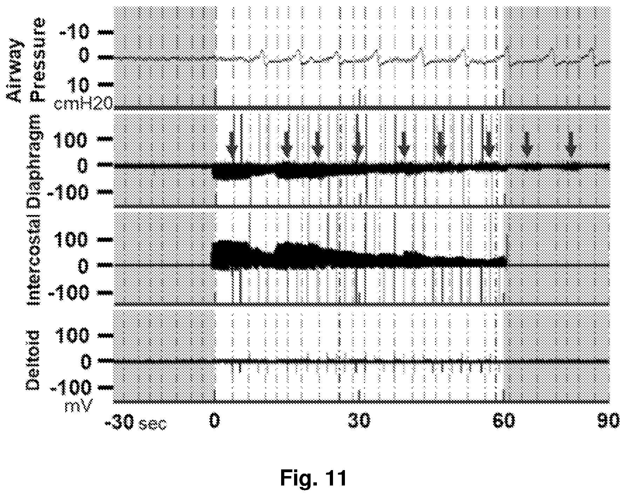

Furthermore, FIG. 11 illustrates transcutaneous electrical stimulation at C2/3 inducing coordinated spontaneous respiratory activity in anesthetized humans. EMG activity was obtained in anesthetized patient (remifentanil and propofol) prior to start of elective surgery. The airway pressure and EMG pattern changes were assessed during control sham-stimulation (not shown) for 2 minutes without evident respiratory and EMG activity. 30 Hz TES was applied at the dorsal neck surface (C2/3 spinal level) with hydrogel electrodes. Constant current stimulation was delivered at the optimal current of 75 mA with a square-wave, 30-Hz, bi-phasic pulse. Prior to stimulation for 30 seconds, there was no evidence of respiratory pressure or muscle EMG activity. During 30 Hz stimulation at the C2/3 level, respiratory activity was evident with bursting activity in diaphragm (delineated by arrow) and in intercostal muscles despite electrical noise caused by stimulation. Moreover, there was no discernable activity from the non-respiratory muscle deltoid. Similar results were observed in 9 other subjects.

Overall, TES of the spinal cord was shown to activate spinal cord motor neurons related to intercostal and diaphragm muscles (FIGS. 9A-9C). TES of the spinal cord was shown to reset the respiratory rhythm and increase both the depth and frequency of breathing during remifentanil-induced respiratory depression (FIGS. 10B, 10C) and induce spontaneous respiratory activity in states without pre-existing respiration (FIG. 10D, FIG. 11). Thus, TES may activate respiratory muscles segmentally when restricted regions of the dorsal cervical spinal cord are targeted using unique stimulation parameters, and TES may activate all respiratory muscles, including those innervated by cranial nerves, when stimulation is sufficient to reset and activate the respiratory CPG in the medulla. It is hypothesized that TES works by 1) activating local neural circuits the elicit segmental motor responses (FIGS. 9A-9C) or 2) by activating rostrally-directed sensory input to brainstem respiratory circuits that in turn increase widespread activation of respiratory muscles (CPG). With these 2 types of stimulation methods, it may be possible to decrease the time on mechanical ventilation by decreasing respiratory muscle atrophy. Given the enormous potential impact of our stimulation protocols to improve respiratory muscle function, we recognize a need to translate this technology to the clinic and marketplace.

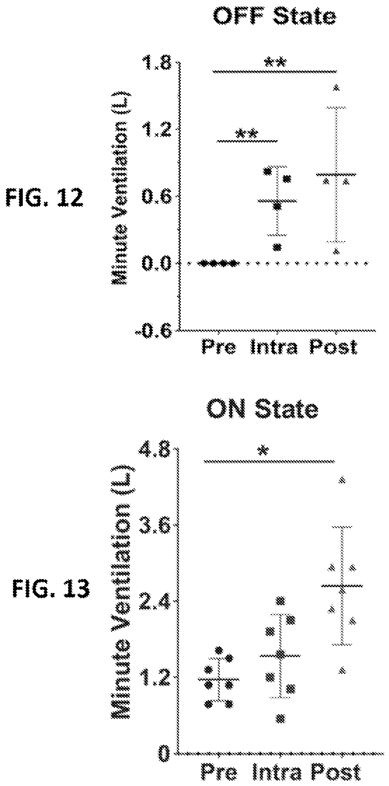

FIG. 12 illustrates transcutaneous electrical stimulation inducing spontaneous respiratory activity in patient states without pre-existing respiration, via CPG activation of respiratory muscles. Specifically, four patients on mechanical ventilation lacked respiratory drive and spontaneous respiration (OFF state). Bilateral, monophasic, 30 Hz transcutaneous electrical stimulation (TES) was applied, targeting upper cervical spinal cord. Minute ventilation (tidal volume x frequency) was assessed in three phases: before simulation ("Pre"), during stimulation ("Intra"), and after stimulation ("Post"). TES induced spontaneous breaths when there was stimulation (Intra, p = 0.00926) with sustained breathing after stimulation (Post, p = 0.00987). P-value of p < 0.01 was calculated by one-way ANOVA and post-hoc Dunnett's multiple comparison test.

FIG. 13 illustrates transcutaneous electrical stimulation inducing activation of respiratory muscles through stimulation providing respiratory central pattern generation. Specifically, TES at cervical spinal cord modulated the respiratory minute tidal volume (L) in a stimulation frequency-dependent manner in seven patients with spontaneous breathing (ON state). Minute ventilation during 30 Hz stimulation ("Intra") or after stimulation ("Post") phases, versus the average of the pre-stimulation ("Pre") were calculated to reflect the respiratory changes induced by TES. The minute ventilation was significantly elevated after the stimulation (30 Hz Post/Pre, p = 0.0277). P-value of p < 0.05 was calculated based on two-way ANOVA and Sidak's multiple comparisons test.

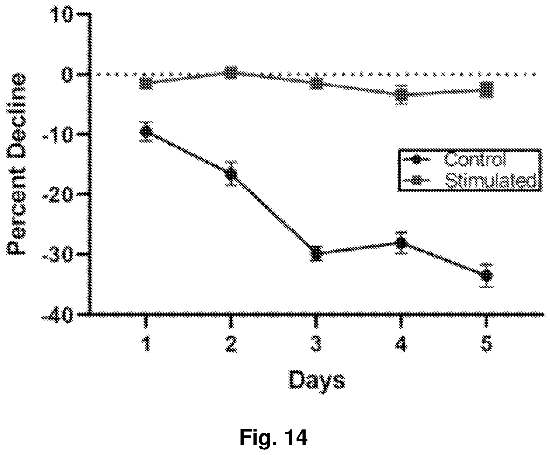

FIG. 14 illustrates transcutaneous electrical stimulation preserving muscle integrity in a mechanically ventilated patient through stimulation providing respiratory central pattern generation. Specifically, a 72 year old patient with COVID and ARDS was intubated for acute respiratory failure and treated with TES daily (one daily for 60 minutes at C3 and T8 locations with 30 Hz, bipolar stimulation gated to be ON during inspiration. The stimulation protocol included dual channel, C3 and T8 bipolar stimulation performed in a closed-loop manner gated to inspiratory phase, at a 30 Hz frequency with sub-motor threshold intensity (75 mA). Assessment of right diaphragm muscle thickness was taken within four hours of initiation of mechanical ventilation and daily for five days (square data point markers). Throughout the five days on mechanical ventilation, there was no evidence of diaphragm muscle thickness decline. In contrast, a control subject (circle data point markers) with a similar clinical profile (68 year old patient with COVID and ARDS) without TES treatment, decrement in respiratory muscle thickness was evident throughout the period of mechanical ventilation with loss of greater than 30% of diaphragm muscle thickness. Thus, compared to the control subject without TES treatment while on mechanical ventilation, the patient treated with TES appeared to benefit, with no evidence of diaphragm muscle atrophy.

### Example 4 - CPG activation of respiratory muscles through epidural electrical stimulation of the lumbar spinal cord.

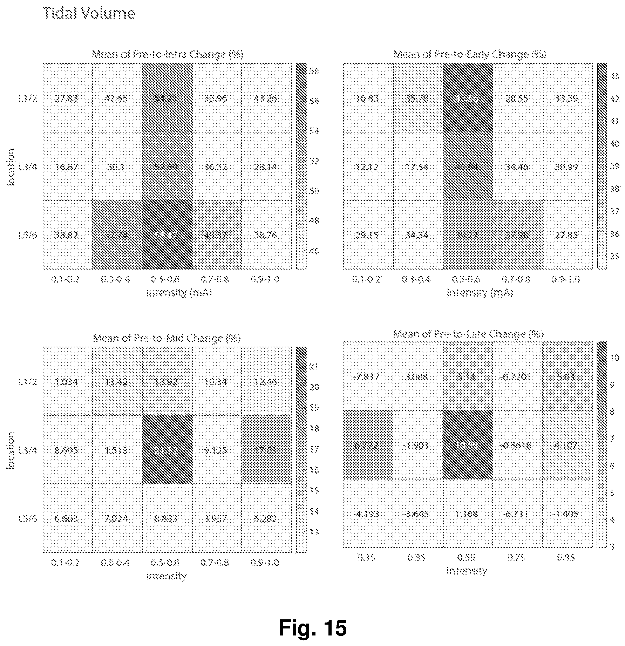

FIGS. 15-17 are plots of epidural electrical stimulation (EES) respiratory responses in mice corresponding to CPG activation of respiratory muscles. A set of EES locations included rostral lumbar (L1L2), middle lumbar (L3L4), and caudal lumbar (L5L6) segments. The stimulation locations were verified by examining corresponding post-mortem tissue. Physiological parameters were measured before, during, and after stimulation. In particular, respiratory pressure was measured, and each of the left and right diaphragms were measured using EMG sensors. Measured signal data was processed to generate estimated respiratory features including exhalation peaks, inhalation troughs, exhalation onsets, and inhalation onsets.

A set of surface plots of phase and stimulation intensity of EES-induced respiratory responses were generated (not shown) for a set of timepoints including pre-stimulation baseline, intra-stimulation, immediately post-stimulation, 3 minutes post-stimulation, and 6 minutes post-stimulation. Furthermore, plots were generated with respect to changes of the tidal volume ratio in response to EES applied at L1L2, L3L4, and L5L6 spinal levels, changes of the frequency ratio in response to EES applied at L1L2, L3L4, and L5L6 spinal levels, changes of the minute ventilation ratio in response to EES applied at L1L2, L3L4, and L5L6 spinal levels. Stimulation was performed at 30 Hz. It should be noted that the L6 spinal level of a mouse is considered to correspond to the L5 spinal level of a human since humans do not have an L6 spinal level.

FIG. 15 illustrates a set of EES-induced tidal volume heatmaps providing respiratory central pattern generation where an average percent change of respiratory parameters compared to a pre-stimulation baseline is plotted and are given by equation 1: $\left(% change=\frac{absolute value - baseline}{baseline}\times 100%\right)$ (equation 1)

In FIG. 15, changes about a predetermined threshold (P<0.05) are darker, whereas changes below the predetermined threshold are lighter. L5/6 provided the most robust change for tidal volume although L5/6 EES was associated with animal movement and muscle contractions such that L1/2 at 0.5mA intensity was selected as a stimulation parameter.

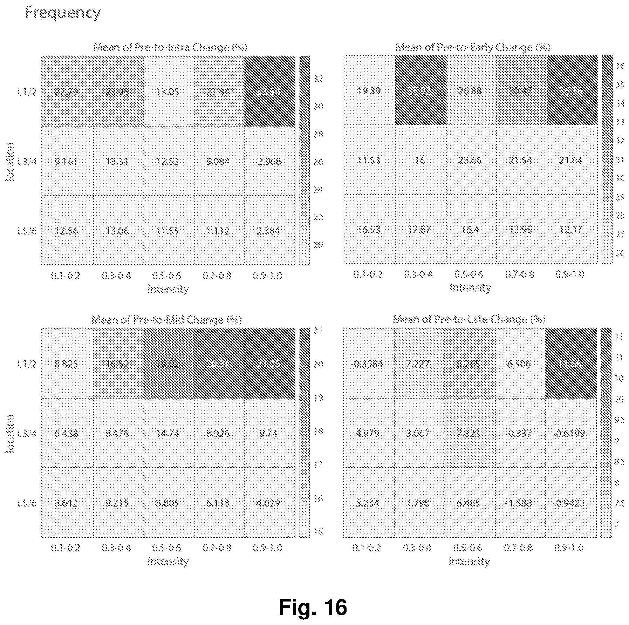

FIG. 16 illustrates a set of EES-induced respiratory frequency heatmaps providing respiratory central pattern generation where an average percent change of respiratory parameters compared to a pre-stimulation baseline is plotted and given by equation 1. In FIG. 16, changes about a predetermined threshold (P<0.05) are darker, whereas changes below the predetermined threshold are lighter. L1/2 EES generated the largest frequency response.

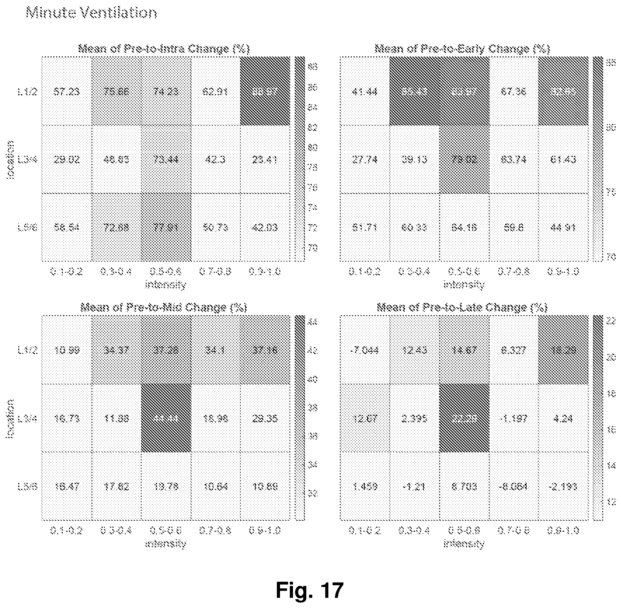

FIG. 17 illustrates a set of EES-induced minute ventilation heatmaps providing respiratory central pattern generation where an average percent change of respiratory parameters compared to a pre-stimulation baseline is plotted and given by equation 1. In FIG. 17, changes about a predetermined threshold (P<0.05) are darker, whereas changes below the predetermined threshold are lighter.

It is noted that the term "patient" used herein can refer to a human or to a non-human mammal under the care of a medical practitioner. However, the methods provided herein can be applied to subjects that are not under the control or care of a medical practitioner. Thus, the terms "subject", "individual," and "patient" may be used interchangeably and typically refer to a mammal, and in certain embodiments a human or a non-human primate. It will be recognized that while the methods are described herein with respect to use in humans, in certain embodiments they are also suitable for animal, e.g., veterinary use. Thus, certain illustrative subjects include, but are not limited to humans, non-human primates, canines, equines, felines, porcines, ungulates, lagomorphs, and the like. Certain embodiments contemplate the methods described herein for use with domesticated mammals (e.g., canine, feline, equine), laboratory mammals (e.g., mouse, rat, rabbit, hamster, guinea pig), and agricultural mammals (e.g., equine, bovine, porcine, ovine), and the like. The term "subject" does not require one to have any particular status with respect to a hospital, clinic, or research facility (e.g., as an admitted patient, a study participant, or the like). Thus, in various embodiments, the subject can be a human (e.g., adult male, adult female, adolescent male, adolescent female, male child, female child) under the care of a physician or other health worker in a hospital, psychiatric care facility, as an outpatient, or other, clinical context. In certain embodiments the subject may not be under the care a physician or health worker and, in certain embodiments, may self-prescribe and/or self-administer the methods provided herein.

The foregoing description, for purposes of explanation, used specific nomenclature to provide a thorough understanding of the invention. However, it will be apparent to one skilled in the art that specific details are not required in order to practice the invention. Thus, the foregoing descriptions of specific embodiments of the invention are presented for purposes of illustration and description. They are not intended to be exhaustive or to limit the invention to the precise forms disclosed; obviously, many modifications and variations are possible in view of the above teachings. The embodiments were chosen and described in order to explain the principles of the invention and its practical applications, they thereby enable others skilled in the art to utilize the invention and various embodiments with various modifications as are suited to the particular use contemplated. It is intended that the following claims define the scope of the invention.

## Claims

1. A system (200) for conditioning muscles in a patient, the system comprising:
a controller (210) configured to detect an inspiratory phase of the patient based on a sensor signal from one or more sensors (240); and
a stimulator (230) configured to administer a stimulation signal to one or more of a cervical, thoracic, and lumbar spinal cord of the patient during the detected inspiratory phase,
wherein the stimulation signal is effective to activate a diaphragm muscle in the patient during the entire detected inspiratory phase, thereby maintaining strength of the diaphragm muscle.

2. The system of claim 1, wherein the one or more sensors comprises a sensor configured to detect chest wall expansion, or wherein at least one of the one or more sensors is coupled to a mechanical ventilator treating the patient.

3. The system of claim 1 or 2, wherein the stimulation signal is superimposed on a high frequency carrier signal.

4. The system of any one of claims 1-3, wherein the stimulator is an electrical stimulator.

5. The system of any one of claims 1-4, wherein the stimulator comprises one or more adhesive stimulators.

6. The system of any one of claims 1-5, further comprising:
a second stimulator configured to administer a second stimulation signal to a cervical spinal cord of the patient, wherein the second stimulation signal is effective to activate respiratory drive in the patient; or
a third stimulator configured to administer a third stimulation signal to a lumbar spinal cord region of the patient.

7. The system of any one of claims 1-6, wherein the controller is further configured to detect an expiratory phase of the patient based on a second sensor signal from one or more sensors, and wherein the stimulator is configured to cease administration of the stimulation signal during the detected expiratory phase.

8. The system of claim 1, wherein the stimulation signal is configured to activate the diaphragm muscle via activating motor neurons at a segmental spinal cord level.

9. The system of claim 1, wherein the stimulator is configured to administer transcutaneous stimulation.

10. The system of claim 1, wherein the stimulator is configured to administer epidural or percutaneous stimulation.

11. The system of claim 1, wherein the stimulator is a magnetic stimulator.

12. The system of claim 1, wherein the patient has a respiratory insufficiency or failure caused by any one or more of: acute respiratory distress syndrome (ARDS), ECMO, ventilator-induced diaphragm dysfunction, critical illness myopathy, chronic obstructive pulmonary disease (COPD), stroke, spinal cord injury, heart failure, trauma, pneumonia, sepsis, aging, and a neurodegenerative disorder.

## Patentansprüche

1. System (200) zum Konditionieren von Muskeln in einem Patienten, wobei das System Folgendes umfasst:
eine Steuerung (210), die ausgelegt ist, um eine Einatmungsphase des Patienten basierend auf einem Sensorsignal von einem oder mehreren Sensoren (240) zu detektieren; und
einen Stimulator (230), der ausgelegt ist, um ein Stimulationssignal an eines oder mehrere aus einem zervikalen, thorakalen und lumbalen Rückenmark des Patienten während der detektierten Einatmungsphase einzuspeisen,
wobei das Stimulationssignal wirksam ist, um einen Zwerchfellmuskel in dem Patienten während der gesamten detektierten Einatmungsphase zu aktivieren, wodurch die Kraft des Zwerchfellmuskels aufrechterhalten wird.

2. System nach Anspruch 1, wobei der eine oder die mehreren Sensoren einen Sensor umfasst, der ausgelegt ist, um eine Brustwandausdehnung zu detektieren, oder wobei zumindest einer aus dem einen oder den mehreren Sensoren mit einem mechanischen Beatmungsgerät, das den Patienten behandelt, gekoppelt ist.

3. System nach Anspruch 1 oder 2, wobei das Stimulationssignal einem Hochfrequenz-Trägersignal überlagert ist.

4. System nach einem der Ansprüche 1 bis 3, wobei der Stimulator ein elektrischer Stimulator ist.

5. System nach einem der Ansprüche 1 bis 4, wobei der Stimulator einen oder mehrere Haft-Stimulatoren umfasst.

6. System nach einem der Ansprüche 1 bis 5, das weiters Folgendes umfasst:
einen zweiten Stimulator, der ausgelegt ist, um ein zweites Stimulationssignal an ein zervikales Rückenmark des Patienten einzuspeisen, wobei das zweite Stimulationssignal wirksam ist, um den Atemantrieb in dem Patienten zu aktivieren; oder
einen dritten Stimulator, der ausgelegt ist, um ein drittes Stimulationssignal an einen lumbalen Rückenmarksbereich des Patienten einzuspeisen.

7. System nach einem der Ansprüche 1 bis 6, wobei die Steuerung ferner ausgelegt ist, um eine Ausatmungsphase des Patienten basierend auf einem zweiten Sensorsignal von einem oder mehreren Sensoren zu detektieren, und wobei der Stimulator ausgelegt ist, um die Abgabe des Stimulationssignals während der detektierten Ausatmungsphase anzuhalten.

8. System nach Anspruch 1, wobei das Stimulationssignal ausgelegt ist, um den Zwerchfellmuskel über das Aktivieren von Motorneuronen auf segmentaler Rückenmarksebene zu aktivieren.

9. System nach Anspruch 1, wobei der Stimulator ausgelegt ist, um eine transkutane Stimulation einzuspeisen.

10. System nach Anspruch 1, wobei der Stimulator ausgelegt ist, um eine epidurale oder perkutane Stimulation einzuspeisen.

11. System nach Anspruch 1, wobei der Stimulator ein magnetischer Stimulator ist.

12. System nach Anspruch 1, wobei der Patient an respiratorischer Insuffizienz oder Versagen leidet, verursacht durch ein beliebiges oder mehrere der Folgenden: akutem Atemnotsyndrom (ARDS), ECMO, durch Beatmung ausgelöster Zwerchfelldysfunktion, Critical-Illness-Myopathie, chronisch-obstruktiver Lungenerkrankung (COPD), Schlaganfall, Rückenmarksverletzung, Trauma, Herzversagen, Pneumonie, Sepsis, Alterung und eine neurodegenerative Störung.

## Revendications

1. Système (200) de conditionnement de muscles chez un patient, le système comprenant :
un dispositif de commande (210) configuré pour détecter une phase inspiratoire du patient sur la base d'un signal de capteur provenant d'un ou de plusieurs capteurs (240) ; et
un stimulateur (230) configuré pour administrer un signal de stimulation à une ou plusieurs parmi une moelle épinière cervicale, thoracique et lombaire du patient pendant la phase inspiratoire détectée,
dans lequel le signal de stimulation est efficace pour activer un muscle de diaphragme chez le patient pendant toute la phase inspiratoire détectée, en maintenant ainsi la force du muscle de diaphragme.

2. Système selon la revendication 1, dans lequel les un ou plusieurs capteurs comprennent un capteur configuré pour détecter une expansion de la paroi thoracique, ou dans lequel au moins un des un ou plusieurs capteurs est couplé à un ventilateur mécanique traitant le patient.

3. Système selon la revendication 1 ou 2, dans lequel le signal de stimulation est superposé à un signal de porteuse haute fréquence.

4. Système selon l'une quelconque des revendications 1 à 3, dans lequel le stimulateur est un stimulateur électrique.

5. Système selon l'une quelconque des revendications 1 à 4, dans lequel le stimulateur comprend un ou plusieurs stimulateurs adhésifs.

6. Système selon l'une quelconque des revendications 1 à 5, comprenant en outre :
un deuxième stimulateur configuré pour administrer un deuxième signal de stimulation à une moelle épinière cervicale du patient, dans lequel le deuxième signal de stimulation est efficace pour activer une commande respiratoire chez le patient ; ou
un troisième stimulateur configuré pour administrer un troisième signal de stimulation à une région de moelle épinière lombaire du patient.

7. Système selon l'une quelconque des revendications 1 à 6, dans lequel le dispositif de commande est en outre configuré pour détecter une phase expiratoire du patient sur la base d'un second signal de capteur provenant d'un ou de plusieurs capteurs, et dans lequel le stimulateur est configuré pour cesser l'administration du signal de stimulation pendant la phase expiratoire détectée.

8. Système selon la revendication 1, dans lequel le signal de stimulation est configuré pour activer le muscle de diaphragme via une activation de motoneurones à un niveau de moelle épinière segmentaire.

9. Système selon la revendication 1, dans lequel le stimulateur est configuré pour administrer une stimulation transcutanée.

10. Système selon la revendication 1, dans lequel le stimulateur est configuré pour administrer une stimulation épidurale ou percutanée.

11. Système selon la revendication 1, dans lequel le stimulateur est un stimulateur magnétique.

12. Système selon la revendication 1, dans lequel le patient présente une insuffisance ou une défaillance respiratoire causée par l'un ou plusieurs parmi : syndrome de détresse respiratoire aiguë (SDRA), ECMO, dysfonctionnement du diaphragme induit par un ventilateur, myopathie due à une maladie grave, maladie pulmonaire obstructive chronique (MPOC), accident vasculaire cérébral, lésion de la moelle épinière, insuffisance cardiaque, traumatisme, pneumonie, septicémie, vieillissement et trouble neurodégénératif.
